# EUROPÄISCHE PATENTSCHRIFT

(11) **EP 2 841 033 B1**
(45) Veröffentlichungstag und Bekanntmachung des Hinweises auf die Patenterteilung: **17.06.2020**
(21) Anmeldenummer: 13720354.3
(22) Anmeldetag: 29.04.2013
(51) Int. Cl.: A61F 5/37, A44B 11/25, A44B 11/28, A61F 5/01

(54) **ORTHESE ZUM FIXIEREN EINES SCHULTERGELENKS**
ORTHOSIS FOR FIXING A SHOULDER JOINT
ORTHÈSE PERMETTANT LA FIXATION D'UNE ARTICULATION DE L'ÉPAULE

(30) Priorität: 27.04.2012 DE 102012009107; 27.04.2012 US 201261639422 P; 17.04.2013 EP 13164211
(43) Veröffentlichungstag der Anmeldung: 04.03.2015
(73) Patentinhaber: BSN medical GmbH, 20253 Hamburg (DE)
(72) Erfinder: GRUNDEN, Jennifer, 22769 Hamburg (DE); SCHMELTZPFENNING, Timo, 21244 Buchholz (DE); BAUER, Joachim, 22589 Hamburg (DE)
(74) Vertreter: Uexküll & Stolberg
(86) Internationale Anmeldenummer: PCT/EP2013/058932
(87) Internationale Veröffentlichungsnummer: WO 2013/160487

(56) Entgegenhaltungen:
- DE-A1-102004 028 604
- DE-C- 188 606
- DE-U1- 20 116 743
- US-A- 5 830 165
- US-A- 6 146 346
- US-A1- 2007 016 121

## Beschreibung

Die vorliegende Erfindung betrifft eine Orthese zur Immobilisierung eines Schultergelenks eines Patienten.

Im Falle von Schultergelenksverletzungen ist es häufig erforderlich, dieses Gelenk mittels einer Orthese zu immobilisieren oder zu fixieren. Eine derartige Orthese ist beispielsweise aus der DE 197 45 705 C1 bekannt. Insbesondere soll eine solche Orthese eine übermäßige Abduktion des Oberarms, also ein seitliches Abspreizen des Arms, sowie eine Ante- und eine Retroversion des Oberarms, d.h. ein Schwenken nach vorne und nach hinten, verhindern.

Bei der aus dem Stand der Technik bekannten Orthese verlaufen zwei Gurte ausgehend von der verletzten Schulter anterior und posterior von dieser Schulter aus in Richtung des Ellenbogens des Arms, der von dem verletzten Schultergelenk ausgeht. An den freien Enden dieser beiden Gurte ist eine Unterarmauflage angebracht, die den Unterarm aufnimmt. Des Weiteren erstreckt sich von dem Ausgangspunkt der zwei über den Oberarm verlaufenden Gurte ein Schultergurt, der posterior zur contralateralen Schulter und von dort anterior nach unten verläuft, um das Handgelenk des Arms zu halten, der von der verletzten Schulter ausgeht. Schließlich ist ein Zirkulargurt vorgesehen, der mit einem ersten Ende an dem Arm des verletzten Schultergelenks an einem Armabschnitt davon am Ellenbogen oder benachbart dazu befestigt ist und posterior um den Patienten zum Handgelenkbereich der Unterarmauflage führt. Bei dieser, aus dem Stand der Technik bekannten Orthese ergeben sich jedoch zwei Probleme.

Zum einen wird die Position des Oberarms, der von dem verletzten Schultergelenk ausgeht, nur unzureichend durch die Orthese festgelegt. Vielmehr sind nach wie vor eine Abduktion sowie eine Ante- und Retroversion, wenn auch in beschränktem Umfang möglich.

Zum anderen hat es sich als für die Patienten außerordentlich schwierig herausgestellt, eine solche Orthese alleine anzulegen, weil der Zirkulargurt nach dem Anlegen der Orthese an den von der verletzten Schulter ausgehenden Arm noch von dem entsprechenden Handgelenk über den Rücken wiederum zum Oberarm geführt und dort fixiert werden muss. Der Zirkulargurt muss also ohne Hinzunahme des Arms der ersten Schulter posterior um den Körper gelegt werden, was eine besondere Schwierigkeit darstellt und von dem Patienten selbst nicht ausgeführt werden kann.

Die US 6,416,346 B beschreibt eine Schulterstütze zur Immobilisierung der Schulter mit einer Gurtanordnung, die am Oberarm eines ersten Arms des Patienten angreift und diesen umschlingt. Hiervon ausgehend erstreckt sich ein Gurt diagonal nach unten über die Frontalebene des Patienten zur Hüfte auf der anderen Körperseite des Patienten und endet in einem Oberschenkelgurt, der den Oberschenkel schlingt. Ein über die Schulter des Patienten verlaufender Schultergurt umfasst eine Schlinge, die das Handgelenk des ersten Arms des Patienten aufnimmt und dieses in Brusthöhe über dem Oberschenkelgurt festhält.

Aus der US 5,830,165 B ist eine Schlinge aus flexiblem Material bekannt, die um den Körper des Trägers gewickelt wird. Das Schlaufenende nimmt das Handgelenk des einen Arms vor der Brust auf. Die Schlaufe wird dann über die Schulter geschlungen, um dann horizontal unter dem Oberarm des einen Arms entlang über die Brust und wieder den Rücken geschlungen zu werden. Der Endabschnitt der Schlinge verläuft horizontal über den Oberarm und wird vor der Brust an einem frontal gelegenen Schlingenabschnitt befestigt.

Die US 2007/0016121 A1 beschreibt ein Gurtsystem bei dem der Ellbogen von einer Manschette umschlungen wird. Die Manschette verläuft diagonal jeweils nach unten zur Vorder- und Rückseite des Patienten, wo sie an einem Burstgurt festgelegt ist.

Die DE 188 606 C bezieht sich auf eine Vorrichtung zum Immobilisieren des Armes. Unterhalb der Schulterblätter wird ein Hauptgurt um den Körper geschnallt. An dem Rückenteil des Hauptgurtes sind zwei Riemen, von denen der eine den Rücken entlang um den Arm der verletzten Seite wieder zum Ausgangspunkt zurückläuft und der andere unter dem Ellenbogen des verletzten Armes entlang nach vorn über die an die Brust gelegte Hand und über die Schulter der gesunden Seite zurück zum Rückenteil des Hauptgurtes geführt ist. Diese Riemen sind dabei an dem Rückenteil des Hauptgurtes befestigt.

Aus der DE 201 16 743 A1 ist eine Bandage zur Ruhigstellung von verletzten Armen-/Schultergeleneken bekannt, die einen Taillengurt umfasst, an dem eine Oberarmmanschette festlegbar ist. Eine Unterarmmanschette ist ebenfalls an dem Taillengurt festlegbar. Ein Schultergurt verläuft von der Unterarmmanschette über die Schulter des Patienten und ist auf der Rückseite an dem Taillengurt festgelegt.

Die DE 10 2004 028 604 A1 beschreibt eine Bandage für den Schulter- und Oberarmbereich und umfasst eine längenverstellbare Unterarmaufnahme zum lösbaren Anbringen am Unterarm eines zu behandelnden Arms sowie zwei daran anschließende Bandabschnitte, wobei der im Bereich eines Ellbogens des Patienten an die Unterarmaufnahme anschließende Bandabschnitt zur Bildung eines Tragegurts von dorsal über die nicht zu behandelnde Schulter nach ventral führbar ist und unter Festlegung einer Winkelung des zu behandelnden Arms fixierbar ist. Der an einem Handbereich der Unterarmaufnahme angeordnete Bandabschnitt ist zur Bildung eines Haltegurts nach dorsal in Richtung zum zu behandelnden Arm führbar und von posterior nach anterior verlaufend um den zu behandelnden Arm schlingbar, wobei der Haltegurt nach der Umschlingung festlegbar ist.

Es ist ausgehend vom Stand der Technik die Aufgabe der vorliegenden Erfindung, eine Orthese zum Immobilisieren eines Schultergelenks bereitzustellen, die das Schultergelenk zuverlässig fixiert, d.h. eine Abduktion sowie eine Ante- oder Retroversion des entsprechenden Arms aus der fixierten Stellung seitlich des Körpers zuverlässig verhindert.

Diese Aufgabe wird gelöst durch eine Orthese gemäß Anspruch 1.

Bei der erfindungsgemäßen Orthese sind demnach ein erster und ein zweiter Gurtabschnitt vorgesehen, die an dem Arm, der von der verletzten Schulter ausgeht, im Bereich eines Armabschnitts, der sich um den Ellenbogen herum erstreckt, in der Weise angreifen, dass sie ihn zumindest teilweise umschlingen. Das zumindest teilweise Umschlingen soll zumindest in dem Umfang erfolgen, dass die fraglichen Gurtabschnitte in der Lage sind, durch Zug in deren Erstreckungsrichtung den Armabschnitt mit einer Kraft zu beaufschlagen. Unter dem Begriff "Armabschnitt" im Sinne der vorliegenden Erfindung ist dabei der Bereich des fraglichen Arms gemeint, der den Ellenbogen selbst sowie die dazu benachbarten Bereiche umfasst.

Der erste Gurtabschnitt übt im angelegten Zustand der Orthese, in der der von der verletzten Schulter ausgehende Arm frontal am Körper anliegt, eine Kraft auf den Armabschnitt aus, die zu dem Körper hin gerichtet ist und die vorzugsweise im Wesentlichen parallel zu der Frontalebene des Patienten verläuft, aber wenigstens eine erste Komponente hat, die horizontal und parallel zur Frontalebene verläuft. Dadurch wird aufgrund des ersten Gurtabschnitts der Armabschnitt an den Körper herangezogen.

Gleichzeitig übt der zweite Gurtabschnitt eine zweite Kraft auf den Armabschnitt aus, die wenigstens eine Komponente hat, die horizontal und parallel zur Sagittalebene und damit senkrecht zu der ersten Komponente verläuft. Vorzugsweise verläuft diese zweite Kraft im Wesentlichen parallel zur Sagittalebene. Vorzugsweise wirkt diese zweite Komponente retrovertierend auf den fraglichen Arm.

Im Unterschied zum Stand der Technik, bei dem nur eine einzige Kraft auf den Armabschnitt ausgeübt wird, wirken zwei Kraftkomponenten auf den Armabschnitt, die zueinander senkrecht stehen, sodass bei der erfindungsgemäßen Orthese erreicht wird, dass der Armabschnitt und damit der sich von der verletzten Schulter erstreckenden Arm sowohl an einer Abduktion als auch an einer Retro- oder Anteversion zuverlässig gehindert ist. Insbesondere wird der Ellenbogen in der Frontalebene im senkrechten Verlauf zu dem Schultergelenk, das zu immobilisieren ist, seitlich am Körper des Patienten fixiert.

Die Erfindung zeichnet sich gegenüber dem Stand der Technik dadurch aus, dass der erste Gurtabschnitt dazu vorgesehen ist, anterior entlang des Körpers des Patienten vom Armabschnitt weg zu verlaufen, und die zweite Komponente ist nach posterior gerichtet, sodass die zweite Kraft eine Retroversion des ersten Arms bewirkt. Dieser Retroversion wirkt jedoch die erste Kraft entgegen, sodass der Oberarm insgesamt gegen den Körper gezogen und fixiert wird. Weiterhin bildet der erste Gurtabschnitt das erste Ende eines Zirkulargurts, der angepasst ist, zirkular und horizontal um den Patienten umzulaufen, wobei das zweite Ende des Zirkulargurts an dem Armabschnitt angreift.

Hierbei ist es zum einen möglich, dass das vom ersten Ende des Zirkulargurts und damit von dem ersten Gurtabschnitt entfernte zweite Ende mit dem erstem Ende verbunden ist, wobei der zweite Gurtabschnitt dann an einem ersten Ende eines Schultergurts der Gurtanordnung ausgebildet ist, der angepasst ist, von dem Armabschnitt posterior zu der zu der ersten Schulter contralateralen Schulter des Patienten zu verlaufen und von der contralateralen Schulter anterior zu dem Zirkulargurt, und wobei das von dem ersten Ende des Schultergurts entfernte zweite Ende auf der anterioren Seite des Patienten in einem ersten Verbindungspunkt mit dem Zirkulargurt verbunden ist. Die erste Kraft wird dann durch den als geschlossenen Ring ausgebildeten Zirkulargurt ausgeübt, während der Zug auf den Schultergurt die zweite Kraft bewirkt.

In diesem Fall ergibt sich der zusätzliche Vorteil, dass durch die Verbindung des zweiten Endes des Schultergurts mit dem Zirkulargurt eine aus Zirkulargurt und Schultergurt gebildete Schlaufe geschaffen wird, in die der Patient nach Anlegen des Armabschnitts nur noch die zur verletzten Schulter contralaterale Schulter einlegen muss, sodass das Anlegen der Orthese stark vereinfacht wird.

Zum anderen gibt es auch die Möglichkeit, dass der zweite Gurtabschnitt an einem von dem ersten Ende entfernten zweiten Ende des Zirkulargurts vorgesehen ist, wobei die Gurtanordnung dann noch einen Schultergurt aufweist, der in einem ersten Verbindungspunkt, der auf der anterioren Seite des Körpers des Patienten liegt, und in einem zweiten Verbindungspunkt, der auf der posterioren Seite des Körpers des Patienten liegt, mit dem Zirkulargurt verbunden ist.

Bei diesem Ausführungsbeispiel werden sowohl die ersten Kraft als auch die zweite Kraft von den Enden des Zirkulargurts auf den Armabschnitt ausgeübt. Der über die contralaterale Schulter laufende Schultergurt übt in diesem Fall keine Kraft auf den Armabschnitt aus. Es ergibt sich aber auch hier der Vorteil, dass der Schultergurt und der Zirkulargurt zusammen eine Schlaufe bilden, in die der Patient die contralaterale Schulter einfach einlegen kann, um die Orthese anzulegen.

Außerdem sorgt bei beiden Alternativen der Schultergurt dafür, dass der Zirkulargurt in seiner horizontalen Position gehalten wird und nicht am Patienten nach unten rutschen kann.

Vorzugsweise sind bei den zuvor beschriebenen Ausführungsformen der erste und der zweite Verbindungspunkt, an dem der Zirkulargurt und der Schultergurt miteinander verbunden sind, entlang des Zirkulargurts verschiebbar. Außerdem kann der erste Verbindungspunkt auch entlang des Schultergurts verschiebbar sein. Insgesamt kann die Orthese dann zum einen einfach an unterschiedliche Patientengrößen angepasst werden und zum anderen lässt sich durch Verschieben der Verbindungspunkte auch die Größe der ersten und zweiten Kraft einstellen.

Des Weiteren kann die erfindungsgemäße Orthese eine Verschlusseinrichtung aufweisen, die eine geöffnete und eine geschlossene Stellung hat, wobei die Gurtanordnung derart ausgestaltet ist, dass in der geschlossenen Stellung die erste Kraft und die zweite Kraft auf den Armabschnitt ausgeübt werden und dass in der geöffneten Stellung weder die erste Kraft noch die zweite Kraft auf den Armabschnitt wirken.

Sofern die Gurtanordnung so bemessen ist, dass sie in der geschlossenen Stellung der Verschlusseinrichtung hinreichend eng am Körper des Patienten anliegt, ist die Verschlusseinrichtung für den Patienten mit dem großen Vorteil verbunden, dass allein durch deren Lösen, also das Überführen von der geschlossenen in die geöffnete Stellung, die Wirkung beider Kräfte aufgehoben wird, sodass der von der verletzten Schulter ausgehende Arm sofort freigegeben ist. Umgekehrt kann allein durch das Schließen der Verschlussvorrichtung sofort die Wirkung der beiden Kräfte erreicht werden. Dies vereinfacht das Anlegen erheblich.

Des weiteren wird darauf hingewiesen, dass das Konzept, bei einer Orthese mit einer Gurtanordnung eine Verschlusseinrichtung einzusetzen, die derart ausgestaltet ist, dass in deren geschlossener Stellung sowohl eine erste als auch eine zweite Kraft auf den Körper, und insbesondere nur einen Körperabschnitt, des Patienten ausgeübt werden, während in der geöffneten Stellung beide Kräfte aufgehoben sind, nicht auf Orthesen zum Fixieren eines Schultergelenks beschränkt ist, sondern bei einer Vielzahl anderer Orthesen zur Anwendung kommen kann.

Bei der erfindungsgemäßen Schulterorthese kann diese Verschlusseinrichtung vorzugsweise an dem ersten Verbindungspunkt vorgesehen sein, an dem der Schultergurt und der Zirkulargurt miteinander verbunden sind und der im angelegten Zustand der Orthese auf der anterioren Seite des Körpers des Patienten liegt. Damit ist die Verschlusseinrichtung für den Patienten gut zugänglich und kann nach dem Einlegen der zu der verletzten Schulter contralateralen Schulter von der geöffneten in die geschlossene Stellung überführt werden, sodass dann die beiden Kräfte auf den Armabschnitt wirken.

Vorzugsweise weist der Zirkulargurt einen Anteriorabschnitt, der sich von dem ersten Verbindungspunkt zu dem ersten Ende des Zirkulargurts erstreckt, und einen Posteriorabschnitt auf, der vorgesehen ist, horizontal und posterior um den Körper des Patienten zu verlaufen, der sich von einem Posteriorabschnittende zu dem zweiten Ende des Zirkulargurts erstreckt und an dem der erste Verbindungspunkt angeordnet ist. Der Anteriorabschnitt und der Posteriorabschnitt können lösbar miteinander verbunden sein. Die Verschlusseinrichtung kann dann vorzugsweise derart ausgestaltet sein, dass in der geschlossenen Stellung der Verschlusseinrichtung der Anteriorabschnitt und der Posteriorabschnitt miteinander verbunden sind und dass in der geöffneten Stellung der Anteriorabschnitt und der Posteriorabschnitt voneinander gelöst sind. Dadurch kann der Zirkulargurt auf der anterioren Seite des Körpers des Patienten aufgetrennt werden, sodass das Anlegen der Orthesen einfach möglich ist. Dabei ist es vorteilhaft, wenn die Verschlusseinrichtung derart ausgestaltet ist, dass der Schultergurt in der geöffneten und der geschlossene Stellung mit dem Posteriorabschnitt verbunden ist. Dann bleibt auch in der geöffneten Stellung die aus dem Schultergurt und einem posterioren Teil des Zirkulargurts gebildete Schlaufe erhalten, die für das Anlegen vorteilhaft ist.

Um ein Spannen des Zirkulargurts zu ermöglichen, weist die die Verschlusseinrichtung eine Fixiervorrichtung zum Festlegen des ersten Verbindungspunkts entlang des Posteriorabschnitts auf, wobei die Fixiervorrichtung derart ausgestaltet ist, dass der erste Verbindungspunkt durch Ziehen an dem Posteriorabschnittende hin zu dem zweiten Ende des Zirkulargurts verschoben werden kann, während eine Bewegung des ersten Verbindungspunkts hin Posteriorabschnittende durch Ziehen an dem zweiten Ende des Zirkulargurts blockiert wird.

Um zu ermöglichen, dass der Unterarm des sich von der verletzten Schulter erstreckenden Arms ebenfalls in einer definierten Stellung gehalten werden kann, weist die Orthese vorzugsweise eine Halteeinrichtung auf, die ausgestaltet ist, den Unterarm in dessen gebeugter Stellung zu halten. Dabei kann die Halteeinrichtung einen Haltegurt aufweisen, der mit einem ersten Ende mit der Gurtanordnung verbunden ist und der angepasst ist, den Unterarm des Arms, der sich von dem ersten Schultergelenk erstreckt, im Bereich von dessen Handgelenk zu halten. Um die Last des Unterarms auf die Gurtanordnung zu übertragen, kann der Haltegurt mit einem ersten Ende mit dem Schultergurt verbunden sein oder dessen erstes Ende wird durch den Schultergurt gebildet. Insbesondere kann der Haltegurt angepasst sein, den Unterarm zu umschlingen, wobei ein zweites Ende des Haltegurts lösbar mit dem Schultergurt verbunden werden kann.

Schließlich kann die erfindungsgemäße Orthese eine Unterarmauflage aufweisen, die angepasst ist, den Unterarm sowie die Hand der des von dem ersten Schultergelenk ausgehenden Arms aufzunehmen. Damit wird verhindert, dass die Hand während des Tragens abknickt, was zu Schäden führen kann.

Auch offenbart wird ein Verschluss mit einem ersten Verschlussteil und einem zweiten Verschlussteil, die lösbar miteinander verbunden werden können, wobei das erste und das zweite Verschlussteil Verbindungseinrichtungen für Gurte der Gurtanordnung aufweisen, wobei das erste Verschlussteil einen Zapfen aufweist, wobei das zweite Verschlussteil ein Anschlussende, an dem die Verbindungseinrichtung ausgebildet ist, und ein Kopplungsende aufweist und zwischen dem Anschlussende und dem Kopplungsende eine sich in einer Verschlussebene erstreckende Führungsbahn vorgesehen ist, die sich von einer Eintrittsöffnung zu einem Führungsbahnende erstreckt und die ausgestaltet ist, den Zapfen aufzunehmen und zwischen der Eintrittsöffnung und dem Führungsbahnende entlang der Führungsbahn zu führen, wobei der Zapfen einen ersten, der Verbindungseinrichtung des ersten Verschlussteils nahen Abschnitt und einen sich an den ersten Abschnitt anschließenden und an dem der Verbindungseinrichtung gegenüberliegenden Ende des ersten Abschnitts angeordneten zweiten Abschnitt aufweist, wobei die Abmessung des ersten Abschnitts senkrecht zur Erstreckungsrichtung des Zapfens der Abmessung der Führungsbahn in der Verschlussebene am Führungsbahnende entspricht und wobei die Abmessungen des zweiten Abschnitts senkrecht zur Erstreckungsrichtung des Zapfens größer ist als die Abmessung der Führungsbahn am Führungsbahnende, sodass der zweite Abschnitt eine Bewegung des Zapfens relativ zu dem zweiten Verschlussteil senkrecht zur Verschlussebene begrenzt, wenn der Zapfen am Führungsbahnende ist.

Ein derartig aufgebauter Verschluss lässt sich auch bei einer angelegten Orthese, wie zum Beispiel bei der erfindungsgemäßen Orthese, mit einer Gurtanordnung einfach durch den Patienten verschließen, da dieser lediglich den Zapfen im Bereich der Eintrittsöffnung in die Führungsbahn einsetzen muss und der Zapfen dann, wenn er entlang der Führungsbahn zu dem Führungsbahnende bewegt worden ist, daran gehindert ist, aus der Führungsbahn gelöst zu werden, die beiden zu verbindenden Enden der Gurtanordnung also gegeneinander verriegelt sind. Da die Abmessungen des zweiten Abschnitts des Zapfen senkrecht zu dessen Erstreckungsrichtung größer sind als die Abmessungen der Führungsbahn am Führungsbahnende, wird eine Bewegung des Zapfens senkrecht zur Ebene der Führungsbahn unabhängig von der Stellung des Zapfens relativ zum Führungsbahnende verhindert.

In einer bevorzugten Ausführungsform des Verschlusses ist die Führungsbahn derart ausgestaltet, dass jede mögliche Bewegung des Zapfens aus dessen Stellung im Führungsbahnende heraus entweder senkrecht zu einer Verbindungslinie zwischen dem Anschlussende und dem Kopplungsende verläuft oder die Projektion dieser Bewegung auf die Verbindungslinie zum Anschlussende weist. Diese Ausgestaltung hat zur Folge, dass bei einer Spannung, die eine Gurtanordnung einer Orthese auf den Verschluss ausübt, der Zapfen am Führungsbahnende gehalten wird.

Wenn also ein derartig aufgebauter Verschluss insbesondere in einer erfindungsgemäßen Orthese verwendet wird, die im verschlossenen Zustand unter einer gewissen Vorspannung steht, die dann zu einer Kraft auf den Verschluss führt, die entlang der Verbindungslinie zwischen dem Anschlussende und dem Kopplungsende verläuft, ergibt sich der folgende Vorteil. Beim Verschließen der Orthese ist es lediglich erforderlich, dass der Zapfen in die entsprechend bemessene Eintrittsöffnung der Führungsbahn eingesetzt und entlang der Führungsbahn zu dem Führungsbahnende bewegt wird, wobei diese Bewegung allein durch die Form der Führungsbahn vorgegeben ist und durch die zuvor beschriebene Kraft erleichtert wird.

Denn wenn der Zapfen beabstandet von dem Führungsbahnende ist, wirkt auf das erste Verschlussteil diese Kraft, die den Zapfen zum Führungsbahnende zieht. Umgekehrt muss der Zapfen entgegen dieser Kraft vom Führungsbahnende wegbewegt werden, wenn die Orthese geöffnet werden soll. Dies gilt auch dann, wenn die Bewegung aus dem Führungsbahnende senkrecht zur Verbindungslinie verläuft, da dann zumindest Reibungskräfte wirken, die bei einer Bewegung aus dem Führungsbahnende heraus überwunden werden müssen. Somit verriegelt sich der erfindungsgemäße Verschluss bei einer Vorspannung in der Gurtanordnung selbst.

In einer weiteren bevorzugten Ausführungsform des Verschlusses sind das Führungsbahnende und der Zapfen derart ausgestaltet, dass der erste Verschlussteil gegenüber dem zweiten Verschlussteil verschwenkbar ist, wenn der Zapfen sich am Führungsbahnende befindet. Dies ermöglicht, dass sich die beiden Teile des Verschlusses entsprechend der auf sie wirkenden Kräfte zueinander ausrichten können. Hier ist es insbesondere möglich, dass der erste Abschnitt einen kreisförmigen Querschnitt aufweist und der Durchmesser des ersten Abschnitts der Weite des Führungsbahnendes, vorzugsweise der gesamten Führungsbahn, entspricht. In diesem Zusammenhang ist unter dem Begriff der Weite die Abmessung der Führungsbahn senkrecht zu deren Verlauf bzw. senkrecht zur jeweiligen Tangente zu verstehen. Wenn die Weite der gesamten Führungsbahn, ggf. mit Ausnahme eines Abschnitts mit einem Rastvorsprung, dem Durchmesser des Zapfens entspricht, ist dieser über die gesamte Länge der Führungsbahn geführt, kann aber verschwenkt und damit zu der wirkenden ausgerichtet werden.

Des Weiteren ist es bei dem Verschluss vorteilhaft, wenn Verriegelungsmittel vorgesehen sind, um den Zapfen in der Führungsbahn, vorzugsweise am Führungsbahnende, zu halten. Insbesondere können die Verriegelungsmittel in der Weise ausgebildet sein, dass in der Führungsbahn ein Rastvorsprung ausgebildet ist, sodass die Weite der Führungsbahn im Bereich des Rastvorsprungs reduziert ist, wobei das zweite Verschlussteil derart ausgestaltet ist, dass der erste Abschnitt des Zapfens bei der Bewegung von der Eintrittsöffnung zum Führungsbahnende und umgekehrt an dem Rastvorsprung entgegen einer elastischen Gegenkraft vorbei bewegt werden muss. Dies verhindert, dass sich der Zapfen aus der Führungsbahn heraus oder sogar vom Führungsbahnende weg hin zu der Eintrittsöffnung bewegen kann, ohne dass entweder auf den Zapfen selbst oder ein Teil des zweiten Verschlussteils eine Kraft ausgeübt werden muss. Der Zapfen ist also am Führungsbahnende in seiner Position auch mechanisch verriegelt.

In vorteilhafter Weise weist das zweite Verschlussteil eine weitere Verbindungseinrichtung für einen weiteren Gurt auf. Damit können an dem zweiten Verbindungsteil mehrere Gurte der Gurtanordnung der Orthese zusammenlaufen.

Ferner ist es bevorzugt, wenn benachbart zur Führungsbahn im Bereich des Führungsbahnendes, vorzugsweise benachbart zur der gesamten Führungsbahn, ein Anlageabschnitt mit einheitlicher Dicke vorgesehen ist, wobei der erste Abschnitt des Zapfens in dessen axialer Richtung eine Länge aufweist, die der Dicke des Anlageabschnitts entspricht. In diesem Fall ist das zweite Verschlussteil zwischen den Vorsprüngen am Zapfen geführt, und die Bewegung des Zapfens entlang der Führungsbahn verläuft ausschließlich in der Ebene des ebenen Abschnitts. Dies ermöglicht eine einfache Verschlussbewegung, die auch von einem in seiner Bewegung eingeschränkten Patienten leicht ausgeführt werden kann.

Ferner kann sich die Führungsbahn bis zum Rand des zweiten Verschlussteils erstrecken, wobei die Eintrittsöffnung im Rand des zweiten Verschlussteils ausgebildet ist. Dann kann das erste Verschlussteil mit dem zweiten in der Weise verbunden werden, dass die Verschlussbewegung ausschließlich in der Ebene des zweiten Verschlussteils erfolgt, das zweite Verschlussteil also wie ein Haken über den Zapfen gezogen wird.

Dabei ist es bevorzugt, wenn die Eintrittsöffnung zwischen dem Anschlussende und dem Kopplungsende auf einer ersten Seite der Verbindungslinie zwischen dem Anschlussende vorgesehen ist, wobei die weitere Verbindungseinrichtung auf einer zweiten, der ersten Seite gegenüberliegenden Seite der Verbindungslinie vorgesehen ist. Auf diese Weise können die beiden Verschlussteile miteinander durch eine Bewegung in der Ebene des ebenen Abschnitts in Eingriff gebracht werden, ohne dass ein an der weiteren Verbindungseinrichtung angebrachter Gurt dabei im Weg ist.

Ferner ist es bei einem solchen Aufbau bevorzugt, wenn die Projektion der Eintrittsöffnung auf der Verbindungslinie zwischen dem Anschlussende und dem Kopplungsende weiter entfernt von dem Kopplungsende ist als die Projektion des Führungsbahnendes auf die Verbindungslinie. Dadurch wird sichergestellt, dass der Zapfen aufgrund der Vorspannung in der Gurtanordnung zum Führungsbahnende und damit weg von der Eintrittsöffnung gezogen wird.

Vorzugsweise verläuft die Führungsbahn mit einer einheitlichen Krümmungsrichtung von der Eintrittsöffnung zu dem Führungsbahnende verläuft, was ein einfaches Verschließen des Verschlusses ermöglicht. Wenn dabei die Führungsbahn am Führungsbahnende derart ausgestaltet ist, dass die Bewegung des Zapfens aus der Stellung am Führungsbahnende heraus parallel zur Verbindungslinie zwischen dem Verbindungsende und dem Kopplungsende verläuft, wird sichergestellt, dass der Zapfen aufgrund der Vorspannung zuverlässig am Führungsbahnende verbleibt.

Schließlich kann in einer bevorzugten Ausführungsform der Zapfen des ersten Verschlussteils derart ausgebildet sein, dass er ein freies Ende aufweist, das mit einer in axialer Richtung des Zapfens verlaufenden Vertiefung versehen ist. Dies ermöglicht, dass der Patient den Zapfen durch Setzen eines Fingers in die Vertiefung in seiner Position festlegen oder bewegen kann, während er mit derselben Hand das zweite Verschlussteil auf den Zapfen führt. Es ist also mit einer Hand möglich, den Verschluss zu verschließen.

Im Folgenden wird die vorliegende Erfindung anhand einer Zeichnung erläutert, die lediglich bevorzugte Ausführungsbeispiele von erfindungsgemäßen Orthesen zeigt, wobei
- Fig. 1: den anterioren Teil eines ersten Ausführungsbeispiels einer Orthese im angelegten und nicht-geschlossenen Zustand zeigt,
- Fig. 2: den anterioren Teil eines ersten Ausführungsbeispiels einer Orthese im angelegten und geschlossenen Zustand zeigt,
- Fig. 3: den posterioren Teil des ersten Ausführungsbeispiels im angelegten Zustand zeigt,
- Fig. 4: den seitlichen Teil des ersten Ausführungsbeispiels im angelegten Zustand zeigt,
- Fig. 5: den anterioren Teil im Bereich des Armabschnitts des ersten Ausführungsbeispiels im angelegten und geschlossenen Zustand zeigt,
- Fig. 6: den anterioren Teil des ersten Ausführungsbeispiels im angelegten und geschlossenen Zustand zeigt, wobei der Arm im flektierten Zustand fixiert ist,
- Fig. 7: den anterioren Teil eines zweiten Ausführungsbeispiels einer Orthese im angelegten und nichtgeschlossenen Zustand zeigt,
- Fig. 8: den anterioren Teil des zweiten Ausführungsbeispiels im angelegten und geschlossenen Zustand zeigt, wobei der Arm nicht fixiert ist,
- Fig. 9: den posterioren Teil des zweiten Ausführungsbeispiels im angelegten und geschlossenen Zustand zeigt,
- Fig. 10: den seitlichen Teil des zweiten Ausführungsbeispiels im angelegten Zustand zeigt,
- Fig. 11: den anterioren Teil des zweiten Ausführungsbeispiels im angelegten und geschlossenen Zustand zeigt, wobei der Arm flektiert aber nicht fixiert ist,
- Fig. 12: das erste Verschlussteil des Verschlusses des dritten Ausführungsbeispiels in perspektivischer Ansicht zeigt,
- Fig. 13: das zweite Verschlussteil des Verschlusses des dritten Ausführungsbeispiels in Draufsicht zeigt,
- Fig. 14: das erste Verschlussteil des Verschlusses des dritten Ausführungsbeispiels in einer Seitenansicht zeigt,
- Fig. 15: eine perspektivische Darstellung des Verschlusses des dritten Ausführungsbeispiels im geschlossenen Zustand zeigt,
- Fig. 16: den anterioren Teil des dritten Ausführungsbeispiels einer Schulterorthese zeigt, wobei die Orthese nicht vollständig geschlossen ist,
- Fig. 17: den posterioren Teil der Orthese aus Fig. 16 zeigt,
- Fig. 18: den seitlichen Teil der Orthese aus Fig. 16 und 17 zeigt,
- Fig. 19: die Orthese aus den Fig. 16 bis 18 mit dem Verschluss in dessen geöffneter Stellung im Detail zeigt,
- Fig. 20: die Orthese aus den Fig. 16 bis 18 mit verschlossenem Verschluss im Detail zeigt,
- Fig. 21: den anterioren Teil der Orthese aus den Fig. 16 bis 18 in vollständig verschlossenem und angelegtem Zustand zeigt,
- Fig. 22: den Ellenbogenabschnitt der Orthese aus den Fig. 16 bis 18 in einer geöffneten Stellung zeigt,
- Fig. 23: den Ellenbogenabschnitt der Orthese aus Fig. 16 bis 18 in einer geschlossenen Stellung zeigt und
- Fig. 24: den Ellenbogenabschnitt der Orthese aus den Fig. 16 bis 18 in einer seitlichen Ansicht zeigt.

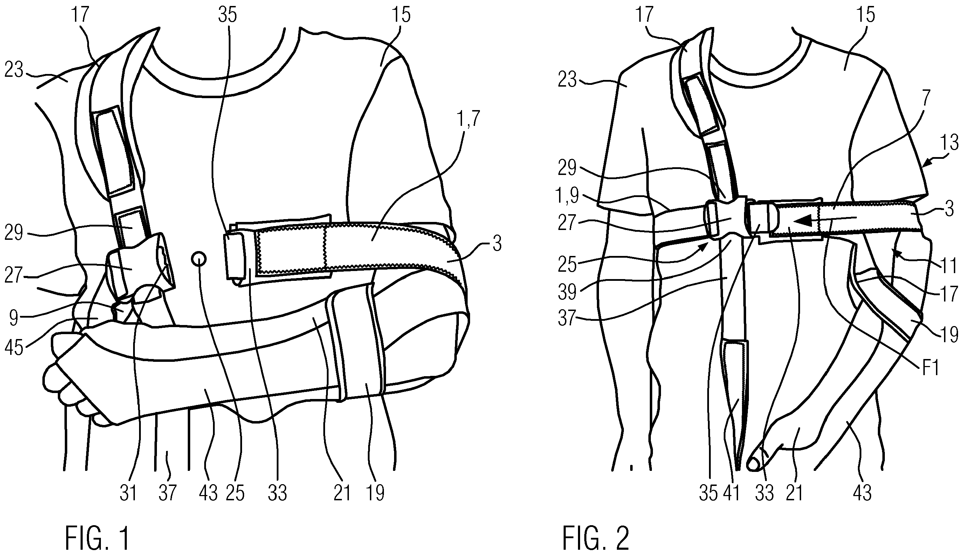

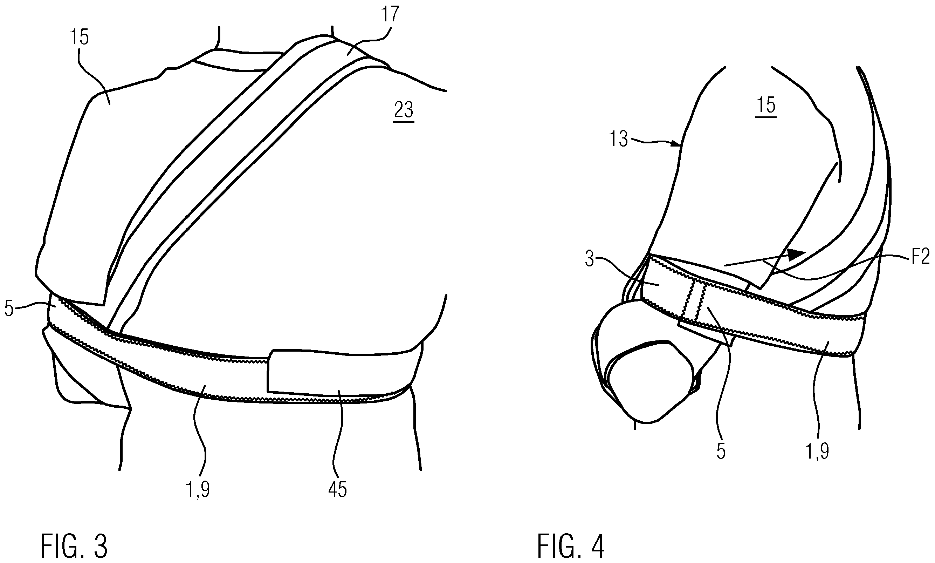

Wie aus den Fig. 1 bis 6 zu erkennen ist, weist das erste Ausführungsbeispiel einer erfindungsgemäßen Orthese zur Immobilisierung eines Schultergelenks eine Gurtanordnung mit einem Zirkulargurt 1 auf, der ein erstes und ein zweites Ende 3, 5 hat (siehe Fig. 4). Der Zirkulargurt 1 setzt sich aus einem sich von dem ersten Ende 3 erstreckenden und anterior über den Patienten verlaufenden Anteriorabschnitt 7 und einem Posteriorabschnitt 9 zusammen, der von dem zweiten Ende 5 posterior und horizontal um den Patienten umläuft. Dabei sind bei diesem Ausführungsbeispiel das erste und das zweite Ende 3, 5 des Zirkulargurts 1 miteinander verbunden bzw. gehen ineinander über. Bei diesem Ausführungsbeispiel bildet das erste Ende 3 des Zirkulargurts 1 einen ersten Gurtabschnitt, der einen Armabschnitt 11 des Arms 13 des Patienten teilweise umschlingt, der sich von dem ruhigzustellenden ersten Schultergelenk 15 erstreckt.

Unter dem Begriff "Armabschnitt" ist dabei der Bereich des von dem verletzten ersten Schultergelenk 15 ausgehenden Arms 13 gemeint, der den Ellenbogen selbst sowie die dazu benachbarten Bereiche umfasst.

Wie insbesondere Fig. 2, 3 und 5 zeigen, ist in diesem Ausführungsbeispiel ein Schultergurt 17 vorgesehen, dessen erstes Ende 19 einen zweiten Gurtabschnitt bildet, wobei das erste Ende 19 den Armabschnitt 11 des Arms 13 des Patienten umschlingt. Dabei liegt das erste Ende 19 an dem Unterarm 21 benachbart zu dem Ellenbogen an. Der Schultergurt 19 erstreckt sich posterior und diagonal über den Rücken des Patienten zu der dem ersten Schultergelenk 15 contralateralen Schulter 23. Wie Fig. 1 zeigt, verläuft der Schultergurt 17 von der contralateralen Schulter 23 in vertikaler Richtung anterior zu dem Zirkulargurt 1 und ist dort in einem ersten Verbindungspunkt mit diesem in bevorzugter Weise lösbar verbunden.

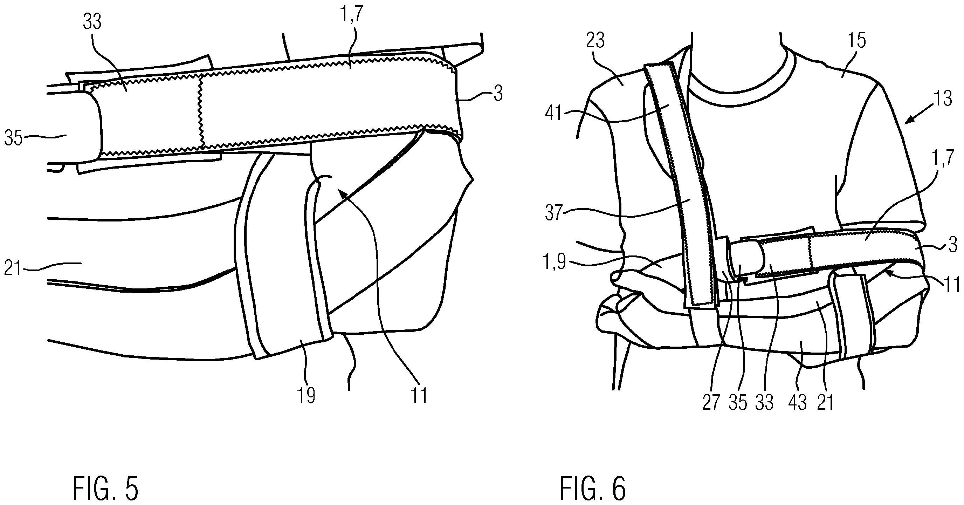

Wie den Fig. 1 und 6 zu entnehmen ist, ist eine Verschlusseinrichtung 27 vorgesehen, die in geschlossenem Zustand der Orthese im Bereich eines virtuellen Knotenpunkts 25 angeordnet ist (siehe Fig. 1 und 2). Die Verschlusseinrichtung 27 ist mit dem zweiten Ende 29 des Schultergurts 17 in der Weise verbunden, dass die Verschlusseinrichtung 27 entlang des Schultergurts 17 verschoben werden kann. Allerdings ist diese Verbindung vorzugsweise derart ausgebildet, dass ein einfaches Hindurchgleiten des Schultergurts 17 durch die Verschlusseinrichtung 27 nicht möglich ist, sondern diese Verbindung ist so ausgestaltet, dass sie den Schultergurt 17 bei Belastung in Verlaufsrichtung Richtung des Schultergurts 17 in einer vorgegeben Position zur Verschlusseinrichtung 27 hält. Zum Verschieben des ersten Verbindungspunktes muss diese Verbindung vorzugsweise zunächst in eine gelöste Stellung gebracht werden. Außerdem ist die Verschlusseinrichtung 27 mit dem Posteriorabschnitt 9 des Zirkulargurts 1 verbunden, wobei sie ebenfalls in dessen Längsrichtung verschiebbar ist. Hierzu ist die Verschlusseinrichtung 27 mit einer im Folgenden noch beschriebenen Fixiervorrichtung versehen. Mittels der Verschlusseinrichtung 27 sind somit das zweite Ende 29 des Schultergurts 17 und der Zirkulargurt 1 bzw. der Posteriorabschnitt 9 miteinander verbunden. Ferner ist dadurch, dass die Verschlusseinrichtung 27 verschiebbar an dem Zirkulargurt 1 und dem Schultergurt 17 angebracht ist, ein erster Verbindungspunkt, an dem der Schultergurt 17 und der Zirkulargurt 1 miteinander verbunden sind, entlang des Zirkulargurts 1 und entlang des Schultergurts 17 verschiebbar.

Die Verschlusseinrichtung 27 ist mit einem Verbindungselement in Form einer Öse 31 versehen, über die das freie Ende 33 des Anteriorabschnitts 7 in der Weise mit den Verschlusseinrichtung 27 verbunden werden kann, dass ein Haken 35 am freien Ende 33 des Anteriorabschnitts 7 in die Öse 31 eingreift. Wenn der Haken 35 in die Öse 31 eingreift, ist die Verschlusseinrichtung 27 in der geschlossenen Stellung und ansonsten in der geöffneten Stellung. Dabei ist zu erkennen, dass sowohl in der geschlossenen als auch in der geöffneten Stellung der Schultergurt 17 und der Posteriorabschnitt 9 miteinander verbunden sind.

Fig. 2 zeigt ferner, dass ein Haltegurt 37 vorgesehen ist, dessen erstes Ende 39 mit dem zweiten Ende 29 des Schultergurts 17 verbunden ist, wobei in diesem bevorzugten Ausführungsbeispiel der Schultergurt 17 und der Haltegurt 37 einstückig ausgebildet sind, sodass der Schultergurt 17 in der Verschlusseinrichtung 27 in den Haltegurt 37 übergeht. Das zweite Ende 41 des Haltegurts 37 kann lösbar mit dem Schultergurt 17 verbunden werden, sodass der Haltegurt 37 eine Schlaufe bildet, in die das Handgelenk des Unterarms 21, der von der dem ersten Schultergelenk 15 ausgeht, aufgenommen werden kann (siehe Fig. 6).

Schließlich ist insbesondere den Fig. 1 und 6 zu entnehmen, dass die Orthese eine Unterarmauflage 43 aufweist, die den Unterarm 21 des von dem ersten Schultergelenk 15 ausgehenden Arms 13 aufnimmt, und die im Armabschnitt 11 dieses Arms 13 mit dem Zirkulargurt 1 und dem Schultergurt 17 verbunden ist. Die Unterarmauflage 43 ist aber für die Funktion der Orthese nicht erforderlich.

Die an der Verschlusseinrichtung 27 angebrachte und nicht im Detail dargestellte Fixiervorrichtung ist vorgesehen, die Lage des ersten Verbindungspunkts, an dem der Schultergurt 17 und der Posteriorabschnitt 9 miteinander verbunden sind, entlang des Posteriorabschnitts 9 festzulegen. Die Fixiervorrichtung ist derart ausgestaltet, dass der erste Verbindungspunkt durch Ziehen an dem vom zweiten Ende 5 des Zirkulargurts 1 bzw. des Posteriorabschnitts 9 entfernten Posteriorabschnittende 45 hin zu dem zweiten Ende 5 verschoben werden kann, während eine Bewegung des ersten Verbindungspunkts hin zum Posteriorabschnittende 45 durch Ziehen an dem zweiten Ende 5 blockiert wird.

Dies bedeutet, dass der Posteriorabschnitt 9 durch Ziehen an dessen Ende 45 durch die Verschlusseinrichtung 27 hindurch gezogen werden kann, während eine Bewegung des Posteriorabschnitts 9 in entgegengesetzter Richtung blockiert ist.

Das zuvor beschriebene erste Ausführungsbeispiel einer Orthese wird wie folgt angelegt.

Zuerst wird der Unterarm 21 in der Unterarmauflage 43 sowie der Armabschnitt 11 in den Enden 3, 19 des Zirkulargurts 1 und des Schultergurts 17 aufgenommen, wobei diese dann den Armabschnitt 5 entweder im Oberarmbereich oder im Unterarmbereich zumindest teilweise umschlingen.

Da das zweite Ende 29 des Schultergurts 17 und der Posteriorabschnitt 9 über die Verschlusseinrichtung 27 auch in deren geöffneter Stellung miteinander verbunden sind, bilden der Posteriorabschnitt 9 und der Schultergurt 17 zunächst eine Schlaufe, in die die zum ersten Schultergelenk 15 contralaterale Schulter 23 aufgenommen werden kann bzw. der Patient muss lediglich diese Schlaufe über die contralaterale Schulter 23 hängen. Danach ergibt sich die in Fig. 1 gezeigte Situation.

Anschließend wird die Verschlusseinrichtung 27 in der Weise geschlossen, dass der Haken 35 am freien Ende 33 des Anteriorabschnitts 7 in die Öse 31 eingesetzt wird. Schließlich kann dann der Zirkulargurt 1 durch Ziehen an dem Posteriorabschnittende 45 gespannt werden, wobei die Verschlusseinrichtung 27 in der zuvor beschriebenen Weise ausgestaltet ist, sodass eine Rückbewegung des Posteriorabschnitts 9 nicht möglich ist. Danach ergibt sich die in Fig. 2 dargestellte Situation, wobei in Fig. 3 zu erkennen ist, dass das Posteriorabschnittende 45 am Posteriorabschnitt 9 befestigt werden kann.

Schließlich kann der Patient den Unterarm 21 in der Weise fixieren, dass der Haltegurt 37 um den Abschnitt der Unterarmauflage 43 geschlungen wird, der das Handgelenk aufnimmt, wobei dann das zweite Ende 41 des Haltegurts 37 an dem Schultergurt 17 oberhalb der Verschlusseinrichtung 27 befestigt wird (siehe Fig. 6).

Bei zuvor beschriebenen Orthese sind demnach ein erster und ein zweiter Gurtabschnitt, nämlich das erste Ende 3 des Zirkulargurts 1 und das erste Ende 19 des Schultergurts 17, vorgesehen, die an dem Armabschnitt 11 in der Weise angreifen, dass sie ihn zumindest teilweise umschlingen, sodass durch Zug in der Erstreckungsrichtung der Gurtabschnitte der Armabschnitt 11 mit einer ersten und einer zweiten Kraft beaufschlagt wird. Der erste Gurtabschnitt bzw. das erste Ende 3 des Zirkulargurts 1 übt im angelegten Zustand der Orthese, in der der von dem verletzten Schultergelenk 15 ausgehende Arm 13 seitlich bzw. frontal am Körper anliegt, eine Kraft (F1; siehe Fig. 2) auf den Armabschnitt 11 aus, die zu dem Körper hin gerichtet ist und die vorzugsweise im Wesentlichen parallel zu der Frontalebene des Patienten verläuft, aber wenigstens eine erste Komponente hat, die horizontal und parallel zur Frontalebene verläuft. Dadurch wird aufgrund des ersten Gurtabschnitts der Armabschnitt 11 an den Körper herangezogen, sodass die erste Kraftkomponente adduzierend auf den ersten Arm 13 wirkt. Gleichzeitig übt der zweite Gurtabschnitt, d.h. das erste Ende 19 des Schultergurts 17 eine zweite Kraft (F2; siehe Fig. 4) auf den Armabschnitt 11 aus, die wenigstens eine Komponente hat, die horizontal und parallel zur Sagittalebene und damit senkrecht zu der ersten Komponente verläuft und nach posterior gerichtet ist, sodass eine leichte Retroversion des Arms 13 bewirkt wird. Aufgrund dieser Kombination der zwei Kraftkomponenten wird der Arm 13 zuverlässig am Körper fixiert.

Außerdem ergibt sich der Vorteil, dass das erste, zu immobilisierende Schultergelenk 15 sowohl bei gestrecktem als auch bei gebeugtem Ellenbogen immobilisiert ist.

Des Weiteren ist die Verschlusseinrichtung 27 für den Patienten mit dem großen Vorteil verbunden, dass allein durch deren Lösen, also das Überführen von der geschlossenen in die geöffnete Stellung, die Wirkung beider Kräfte F1, F2 auf den Armabschnitt 11 aufgehoben wird, sodass der von dem verletzten Schultergelenk 15 ausgehende Arm 13 sofort freigegeben ist. Umgekehrt kann allein durch das Schließen der Verschlussvorrichtung 27 sofort die Wirkung der beiden Kräfte F1, F2 erreicht werden, ohne dass weitere Maßnahmen durch den Patienten erforderlich sind. Dies vereinfacht das Anlegen erheblich.

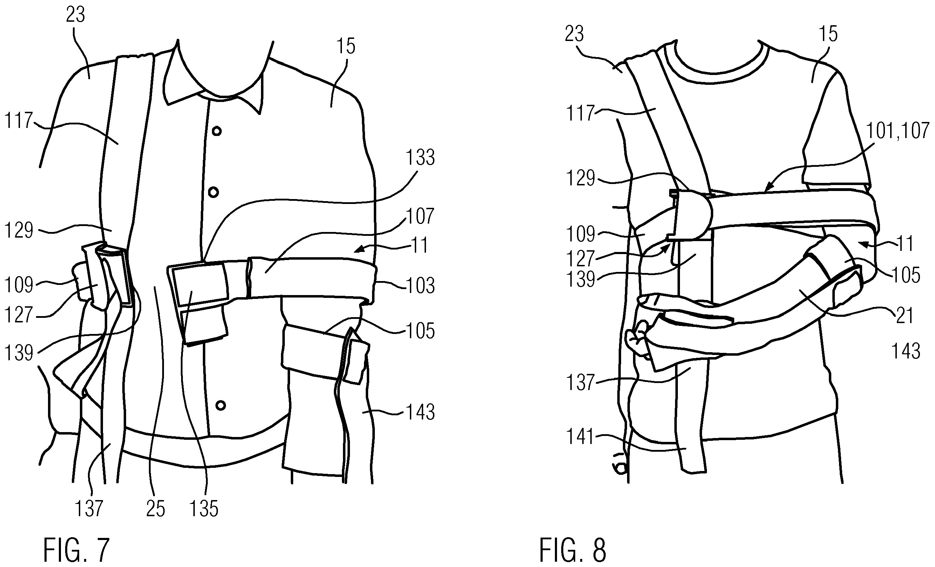

In den Fig. 7 bis 11 ist ein zweites Ausführungsbeispiel einer erfindungsgemäßen Orthese in unterschiedlichen Stadien des Anlegens gezeigt. Dieses Beispiel weist ebenfalls einen Zirkulargurt 101 auf, der ein erstes und ein zweites Ende 103, 105 hat. Der Zirkulargurt 101 umfasst ebenfalls einen sich von dem ersten Ende 103 erstreckenden und anterior über den Patienten verlaufenden Anteriorabschnitt 107 und einen Posteriorabschnitt 109, der von dem zweiten Ende 105 posterior und horizontal um den Patienten herumläuft.

Bei diesem Ausführungsbeispiel bildet das erste Ende 103 des Zirkulargurts 101 einen ersten Gurtabschnitt, der den Armabschnitt 11 des Arms 13 des Patienten umschlingt, wobei dies benachbart zu dem Ellenbogen am Oberarm erfolgt. Das zweite Ende 105 des Zirkulargurts 101 umschlingt ebenfalls den Armabschnitt 11, wobei dies allerdings am Unterarm 21 erfolgt.

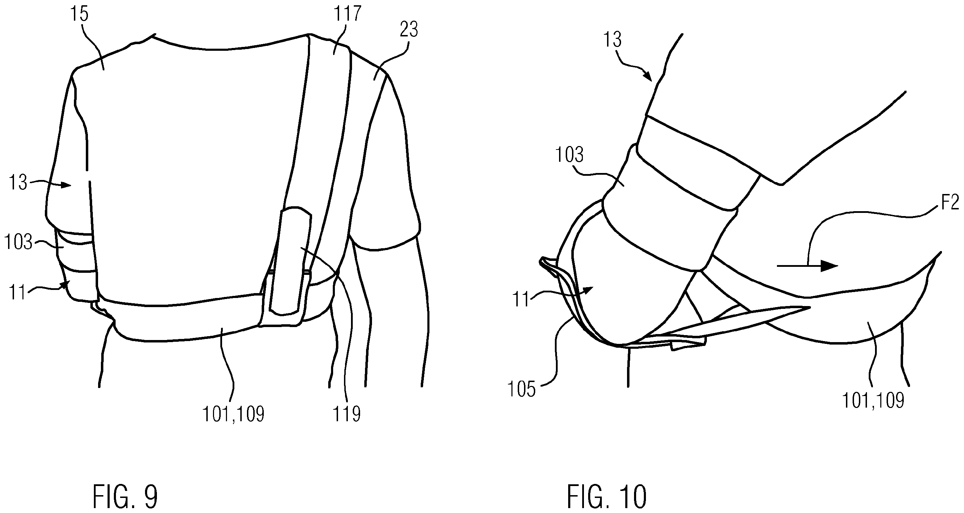

Des Weiteren weist dieses Ausführungsbeispiel einen Schultergurt 117 auf, der bei diesem Ausführungsbeispiel nicht an dem Armabschnitt 11 angelenkt ist. Vielmehr ist dessen erstes Ende 119 mit dem Posteriorabschnitt 109 des Zirkulargurts 101 verbunden ist (siehe Fig. 9). Die Verbindung zwischen dem ersten Ende 119 des Schultergurts 117 und dem Zirkulargurt 101 ist dabei so ausgestaltet, dass das erste Ende 119 den Zirkulargurt 101 umschlingt, sodass der zweite Verbindungspunkt, an dem das erste Ende 119 mit dem Zirkulargurt 101 bzw. dem Posteriorabschnitt 109 verbunden ist, entlang des Zirkulargurts 101 bzw. des Posteriorabschnitts 109 vorzugsweise verschiebbar ist. Dadurch kann die Position des ersten Endes 119 des Schultergurts 117 eingestellt werden. Der Schultergurt 117 erstreckt sich über die zu dem ersten, ruhigzustellenden Schultergelenk 9 contralaterale Schulter 21 hinweg zu dem virtuellen Knotenpunkt 25.

Auch bei diesem Ausführungsbeispiel verläuft der Anteriorabschnitt 107 anterior über den Körper des Patienten hin zu dem virtuellen Knotenpunkt 25, wobei das freie Ende 133 des Anteriorabschnitts 107 mit einem Klettverschlusselement 135 versehen ist. Auch wenn im Folgenden Klettverschlusselemente zur Verbindung von Anteriorabschnitt 107 und Posteriorabschnitt 109 bzw. Verschlusseinrichtung 127 beschrieben werden, können an dieser Stelle auch andere Mittel zum Herstellen einer lösbaren Verbindung eingesetzt werden.

Wie den Fig. 8 und 10 zu entnehmen ist, weist auch dieses Ausführungsbeispiel eine Verschlusseinrichtung 127 auf, die in geschlossenem Zustand der Orthese im Bereich des virtuellen Knotenpunkts 25 liegt (siehe Fig. 8). Die Verschlusseinrichtung 127 ist so mit dem zweiten Ende 129 des Schultergurts 117 verbunden, dass die Verschlusseinrichtung 127 entlang des Schultergurts 117 verschoben werden kann. Auch hier ist diese Verbindung vorzugsweise derart ausgebildet, dass ein einfaches Hindurchgleiten des Schultergurts 117 durch die Verschlusseinrichtung 127 nicht möglich ist. Diese Verbindung ist vielmehr so ausgestaltet, dass sie den Schultergurt 117 bei Belastung in Verlaufsrichtung Richtung des Schultergurts 117 in einer vorgegeben Position zur Verschlusseinrichtung 127 hält. Zum Verschieben des ersten Verbindungspunktes muss diese Verbindung vorzugsweise zunächst in eine gelöste Stellung gebracht werden. Außerdem ist die Verschlusseinrichtung 127 mit dem Posteriorabschnitt 109 des Zirkulargurts 1 verbunden, wobei sie auch in dessen Längsrichtung verschiebbar ist. Hierzu ist die Verschlusseinrichtung 127 mit einer im Folgenden noch beschriebenen Fixiervorrichtung versehen. Mittels der Verschlusseinrichtung 127 sind somit auch hier das zweite Ende 129 des Schultergurts 117 und der Zirkulargurt 101 bzw. der Posteriorabschnitt 109 miteinander verbunden. Ferner ist dadurch, dass die Verschlusseinrichtung 127 verschiebbar an dem Zirkulargurt 101 und dem Schultergurt 117 angebracht ist, ein erster Verbindungspunkt, an dem der Schultergurt 117 und der Zirkulargurt 101 bzw. der Posteriorabschnitt 109 miteinander verbunden sind, entlang des Zirkulargurts 101 bzw. des Posteriorabschnitts 109 und entlang des Schultergurts 117 verschiebbar.

In diesem Ausführungsbeispiel ist die Verschlusseinrichtung 127 mit einem Verbindungselement in Form eines Klettverschlusselements 138 versehen, das mit dem Klettverschlusselement 135 am freien Ende 133 des Anteriorabschnitts 107 lösbar verbunden werden kann, sodass auch die Verschlusseinrichtung 127 eine geschlossene Stellung, in der das freie Ende 133 des Anteriorabschnitts 107 und der Posteriorabschnitt 109 miteinander verbunden sind, und eine geöffnete Stellung hat, in der der das freie Ende 133 des Anteriorabschnitts 107 und der Posteriorabschnitt 109 voneinander gelöst sind.

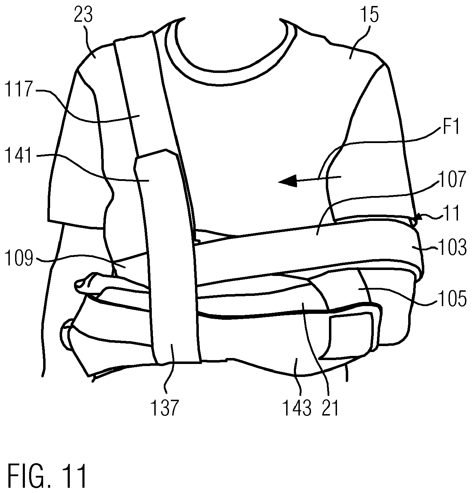

Insbesondere Fig. 8 zeigt, dass auch hier eine Halteeinrichtung für den Unterarm in Form eines Haltegurts 137 vorgesehen ist, dessen erstes Ende 139 mit dem zweiten Ende 129 des Schultergurts 117 hier dadurch verbunden ist, dass der Schultergurt 117 und der Haltegurt 137 einstückig ausgebildet sind, sodass der Schultergurt 117 in der Verschlusseinrichtung 127 in den Haltegurt 137 übergeht. Das zweite Ende 141 des Haltegurts 137 kann lösbar über eine Klettverbindung mit dem Schultergurt 117 verbunden werden, sodass durch den Haltegurt 137 eine Schlaufe gebildet werden kann, in die das Handgelenk des Unterarms 21, der von dem ersten Schultergelenk 15 ausgeht, aufgenommen werden kann (siehe Fig. 11).

Schließlich ist insbesondere den Fig. 8 und 11 zu entnehmen, dass auch bei diesem Ausführungsbeispiel einer erfindungsgemäßen Orthese eine Unterarmauflage 143 vorgesehen ist, wobei eine solche Auflage aber nicht notwendig erforderlich ist. Die Unterarmauflage 143 nimmt den Unterarm 21 des Arms 13 auf und ist im Armabschnitt 11 dieses Arms 13 mit dem zweiten Ende 105 des Zirkulargurts 101 verbunden.

Die an der Verschlusseinrichtung 127 angebrachte und auch hier nicht im Detail dargestellte Fixiervorrichtung ist vorgesehen, die Lage des ersten Verbindungspunkts, in dem der Schultergurt 117 und der Posteriorabschnitt 109 miteinander verbunden sind, entlang des Posteriorabschnitts 109 festzulegen. Die Fixiervorrichtung ist derart ausgestaltet, dass sie eine Verriegelungsstellung und eine Freigabestellung einnehmen kann. In der Verriegelungsstellung kann der erste Verbindungspunkt durch Ziehen an dem vom zweite Ende 105 des Zirkulargurts 101 bzw. des Posteriorabschnitts 109 entfernten Posteriorabschnittende 145 hin zu den zweiten Ende 105 verschoben werden, während eine Bewegung des ersten Verbindungspunkts hin zum Posteriorabschnittende 145 durch Ziehen an dessen zweitem Ende 105 blockiert wird. Anders ausgedrückt kann der Posteriorabschnitt 109 durch Ziehen an dessen Ende 145 durch die Verschlusseinrichtung 127 hindurch gezogen werden kann, während eine Bewegung des Posteriorabschnitts 109 in der entgegengesetzten Richtung durch die Fixiervorrichtung blockiert ist, wenn diese in der Verriegelungsstellung ist. In der Freigabestellung hingegen ist eine Bewegung des ersten Verbindungspunkts hin zum Posteriorabschnittende 145 durch Ziehen an dem zweiten Ende 105 des Zirkulargurts 101 bzw. des Posteriorabschnitts 109 möglich. Dies bedeutet, dass in der Freigabestellung der Posteriorabschnitt 109 durch die Verschlusseinrichtung 127 hindurch gezogen werden kann, sodass sich dessen Ende 145 zur Verschlusseinrichtung 127 hin bewegt.

Das zuvor beschriebene zweite Ausführungsbeispiel einer erfindungsgemäßen Orthese wird in der folgenden Weise angelegt.

Zunächst legt der Patient den Unterarm 21 in die Unterarmauflage 143 ein, und der Armabschnitt 11 wird in den schlaufenförmig gebildeten ersten Enden 103, 105 des Zirkulargurts 101 aufgenommen, wobei das erste Ende 103 des Zirkulargurts 101 den Armabschnitt 11 im Oberarmbereich und das zweite Ende 105 den Unterarmbereich umschlingt.

Da das erste Ende 119 des Schultergurts 117 mit dem Posteriorabschnitt 109 des Zirkulargurts 101 verbunden ist und das zweite Ende 129 des Schultergurts 15 und der Zirkulargurt 1 über die Verschlusseinrichtung 127 unabhängig von deren Stellung ebenfalls miteinander verbunden sind, bilden der Zirkulargurt 101 und der Schultergurt 117 zusammen eine Schlaufe, in die die zum ersten Schultergelenk 15 contralaterale Schulter 23 aufgenommen werden kann bzw. der Patient muss lediglich diese Schlaufe über die contralaterale Schulter 23 hängen. Dann ergibt sich die in den Fig. 7 dargestellte Situation.

Anschließend wird die Verschlusseinrichtung 127 in die geschlossene Stellung gebracht, indem das freie Ende 133 des Anteriorabschnitts 107 in der Weise festgelegt wird, dass die Klettverschlusselemente 135, 138 verbunden werden. Schließlich kann dann der Zirkulargurt 101 durch Ziehen an dem Posteriorabschnittende 145 gespannt werden, wobei die Fixiervorrichtung der Verschlusseinrichtung 127 in der Verriegelungsstellung ist, sodass eine Rückbewegung des Zirkulargurts 101 nicht möglich ist. Das Spannen kann damit allein mittels des Arms erfolgen, der von der contralateralen Schulter 23 ausgeht. Danach ergibt sich die in Fig. 8 und 9 dargestellte Situation.

Schließlich kann der Patient den Unterarm 21 in der Weise am Körper fixieren, dass der Haltegurt 137 um den Abschnitt der Unterarmauflage 143 geschlungen wird, der das Handgelenk aufnimmt, wobei dann das zweite Ende 141 des Haltegurts 137 oberhalb der Verschlusseinrichtung 127 an dem Schultergurt 117 befestigt wird (siehe Fig. 11).

Auch bei diesem zweiten Ausführungsbeispiel kann der Zirkulargurt 1 in einfacher Weise gespannt werden, wobei der von dem ersten Schultergelenk 15 ausgehende Arm 13 fest fixiert ist. Auch hier üben zwei Gurtabschnitte, nämlich das erste und das zweite Ende 103, 105 des Zirkulargurts 101 zwei Kräfte auf den Armabschnitt 11 aus, sodass dieser zuverlässig fixiert wird. Das erste Ende 103 des Zirkulargurts 1 übt im angelegten Zustand der Orthese, eine Kraft auf den Armabschnitt 11 aus, die zu dem Körper hin gerichtet ist und die vorzugsweise im Wesentlichen horizontal und parallel zu der Frontalebene des Patienten verläuft, aber wenigstens eine erste Komponente hat, die horizontal und parallel zur Frontalebene verläuft. Daneben übt das zweite Ende 105 des Zirkulargurts 101 eine zweite Kraft auf den Armabschnitt 11 aus, die wenigstens eine Komponente hat, die parallel zur Sagittalebene und damit senkrecht zu der ersten Komponente verläuft und nach posterior gerichtet ist. Darüber hinaus ist auch hier das erste Schultergelenk 15 sowohl bei gestrecktem als auch bei gebeugtem Ellenbogen des Arms 13 fixiert.

Schließlich ist auch hier die Verschlusseinrichtung 127 vorteilhaft so angeordnet, dass allein durch das Überführen von der geschlossenen in die geöffnete Stellung, also das Lösen der Klettverschlusselemente 135, 138, die Wirkung beider Kräfte auf den Armabschnitt 11 aufgehoben wird, sodass der von dem verletzten Schultergelenk 15 ausgehende Arm 13 sofort freigegeben ist.

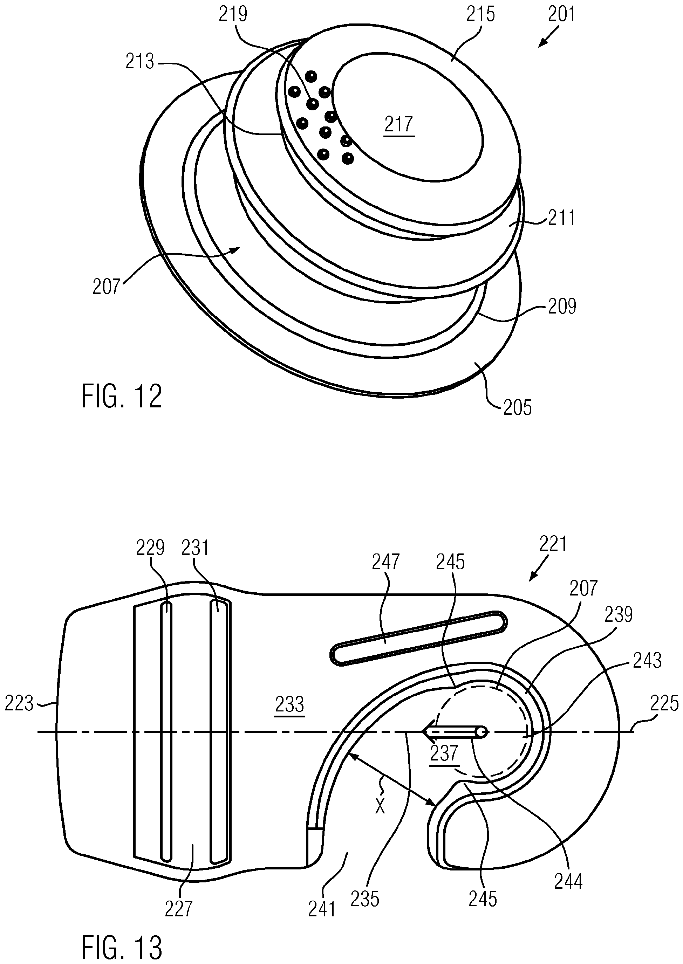

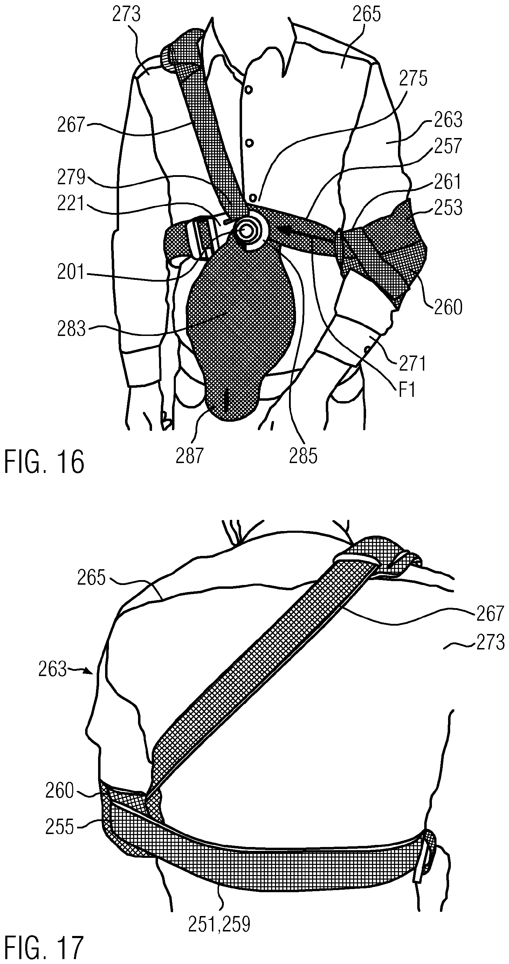

In den Fig. 12 bis 15 ist zunächst der Verschluss eines dritten Ausführungsbeispiels einer erfindungsgemäßen Orthese zur Immobilisierung eines Schultergelenks dargestellt, ohne dass dieser mit der Gurtanordnung der Orthese verbunden ist, während die Fig. 16 bis 24 das dritte Ausführungsbeispiel selbst zeigen.

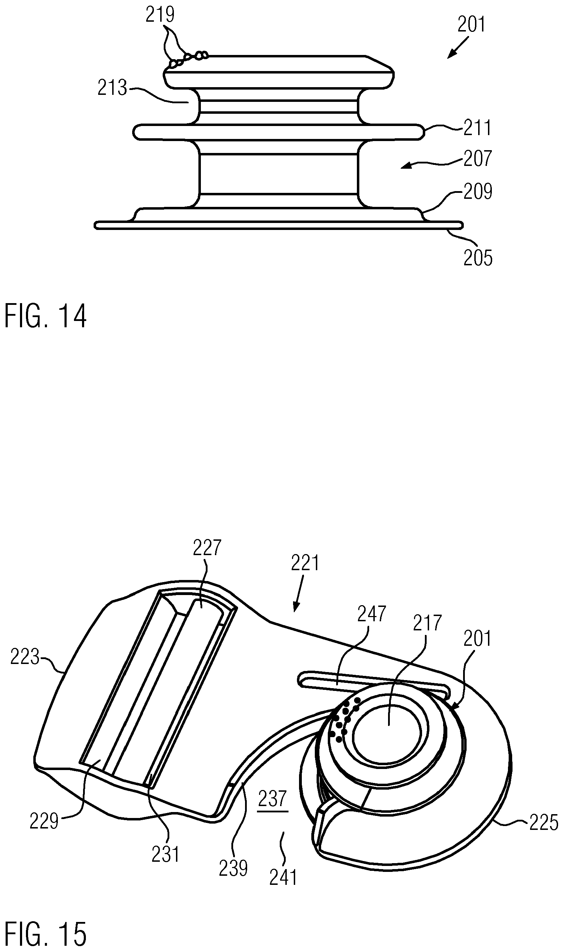

Das in den Fig. 12 und 14 dargestellte erste Verschlussteil 201 des Ausführungsbeispiels umfasst einen Zapfen 203 und weist eine als Flansch 205 ausgebildete Verbindungseinrichtung auf, über den es mit einem Ende eines Gurts einer Gurtanordnung beispielsweise durch Verschweißen, Verkleben oder Vernähen verbunden werden kann. Der Zapfen 203 des ersten Verschlussteils 201 ist mit einem ersten Abschnitt 207 versehen, der sich zwischen einem ersten Vorsprung 209 und einem als zweiten Vorsprung 211 ausgebildeten zweiten Abschnitt erstreckt. Damit ist der erste Abschnitt 207 nahe der als Flansch 205 ausgebildeten Verbindungseinrichtung angeordnet, und der zweite Abschnitt bzw. der zweite Vorsprung 211 schließt sich an dem der Verbindungseinrichtung bzw. dem Flansch 205 gegenüberliegenden Ende des ersten Abschnitts 207 an diesen an. Der Durchmesser und damit die Abmessungen der Vorsprünge 209, 211 senkrecht zur Erstreckungsrichtung des Zapfens 203 sind größer als der Durchmesser bzw. die Abmessungen des zylindrischen ersten Abschnitts 207.

Auf der von dem Flansch 205 abgewandten Seite des ersten Abschnitts 207 ist an dem Zapfen 203 ein Hinterschnitt 213 vorgesehen, der dazu dient, eine Aussparung in einem Gurtende aufzunehmen. Der Hinterschnitt 213 am freien Ende des Zapfens 203 stellt somit eine weitere Verbindungseinrichtung dar, über die beispielsweise ein weiterer Gurt lösbar mit dem ersten Verschlussteil 201 verbunden werden kann. Wie schließlich aus Fig. 1 zu erkennen ist, weist das von dem Flansch 205 abgewandte freie Ende 215 des Zapfens 203 eine sich in axialer Richtung des Zapfens 203 erstreckende Vertiefung 217 auf, die so bemessen ist, dass sie den Finger eines Patienten aufnehmen kann und das erste Verbindungsteil 201 somit in Position gehalten werden kann, wenn der Verschluss verschlossen wird. Um dabei das erste Verschlussteil 201 zusätzlich sicher halten zu können, sind an der Endfläche des freien Endes 215 um die Vertiefung 217 herum eine Vielzahl von Vorsprüngen 219 vorgesehen, die zusätzlich ein Verrutschen des Fingers des Patienten verhindern.

In Fig. 13 ist das zweite Verschlussteil 221 in Draufsicht dargestellt. Das zweite Verschlussteil 221 weist ein Anschlussende 223 sowie ein Kopplungsende 225 auf. Im Bereich des Anschlussendes 223 ist eine erste Verbindungseinrichtung für ein Gurtende vorgesehen, die in diesem Ausführungsbeispiel derart ausgebildet ist, dass sich parallel zu einem Steg 227 ein erster Schlitz 229 und ein zweiter Schlitz 231 erstrecken, wobei der zweite Schlitz 231 weiter von dem Anschlussende 223 entfernt ist als der erste Schlitz 229. Um ein Gurtende mit der ersten Verbindungseinrichtung zu verbinden, wird dieses vom Anschlussende 223 aus entlang einer Seite des zweiten Verbindungsteils 221 und zunächst durch den zweiten Schlitz 231, dann um den Steg 227 herum und schließlich durch den ersten Schlitz 229 hindurch wieder zu dem Anschlussende 223 geführt. Dadurch, dass der erste Schlitz 229 auf der von dem Steg 227 abgewandten Seite scharfkantig begrenzt ist, kann ein Gurtende somit in sich selbst fixierender Weise mit der ersten Verbindungseinrichtung verbunden werden, wobei die Position des Gurtendes relativ zu dem zweiten Verbindungsteil 221 einstellbar ist.

Das zweite Verschlussteil 221 weist ferner einen ebenen Abschnitt 233 auf, der sich von dem Anschlussende 223 bzw. der ersten Verbindungseinrichtung 227, 229, 231 weg hin zu dem Kopplungsende 225 erstreckt. Der ebene Abschnitt 233 erstreckt sich dabei entlang einer Verbindungslinie 235 zwischen dem Anschlussende 223 und dem Kopplungsende 225. In dem ebenen Abschnitt 233 ist eine Führungsbahn 237 als Aussparung ausgebildet, die von einem Steg 239 mit konstanter Dicke ausgebildetem Anlageabschnitt umgeben ist, sodass der ebene Abschnitt 233 im Bereich um die Führungsbahn 237 mit einer einheitlichen Dicke ausgebildet ist. Durch den Steg 239 entspricht die Dicke des ebenen Abschnitts 233 im Bereich der Führungsbahn 237 dem Abstand zwischen dem ersten und zweiten Vorsprung 209, 211 bzw. der Länge des ersten Abschnitts 207 in axialer Richtung des Zapfens 203, sodass das erste Verschlussteil 201, wenn es sich in der Führungsbahn 237 befindet, sich nicht in axialer Richtung des Zapfens 203 relativ zu dem zweiten Verschlussteil 221 bewegen kann. Außerdem erstreckt sich die Führungsbahn 237 in einer durch den ebenen Abschnitt 233 definierten Verschlussebene.

Die Führungsbahn 237 hat einen einheitlich gekrümmten Verlauf und erstreckt von einer Eintrittsöffnung 241 zu einem Führungsbahnende 243, wobei in diesem bevorzugten Ausführungsbeispiel die Eintrittsöffnung 241 am Rand des ebenen Abschnitts 233 und damit des zweiten Verschlussteils 221 ausgebildet ist. Die Eintrittsöffnung 241 ist unabhängig von ihrer Lage im zweiten Verschlussteil 221 derart ausgestaltet, dass durch sie das erste Verschlussteil 201 in die Führungsbahn 237 eingeführt werden kann. Wenn die Eintrittsöffnung 241 im Rand ausgebildet ist, kann das zweite Verschlussteil 221 einfach auf den ersten Abschnitt 209 des Zapfens 203 aufgeschoben werden. Es ist aber auch denkbar, dass die Eintrittsöffnung als Bohrung mit gegenüber dem Führungsbahnende 243 vergrößerten Abmessungen ausgeführt ist, sodass zunächst der zweite Abschnitt bzw. der zweite Vorsprung 211 durch die Bohrung geschoben werden können, bevor dann der erste Abschnitt 207 in der Führungsbahn 237 verschoben wird, wobei der Rand der Führungsbahn 237 dann an dem ersten Abschnitt 207 anliegt.

Die Führungsbahn 237 ist damit derart ausgestaltet, dass jede mögliche Bewegung 244 des Zapfens 203 aus dessen Stellung im Führungsbahnende 243 so verläuft, dass zumindest die Projektion dieser Bewegung 244 auf die Verbindungslinie 235 zum Anschlussende 223 weist, wobei die Lage des ersten Abschnitts 207 des Zapfens 203 im Führungsbahnende 243 in Fig. 13 gestrichelt angedeutet ist. In dem hier gezeigten Ausführungsbeispiel verläuft die Bewegung 244 des Zapfens 203 selbst parallel zur Verbindungslinie 235. Es ist aber auch denkbar, dass die Bewegung geneigt zu der Verbindungslinie verläuft. In jedem Fall ist die Führungsbahn 237 am Führungsbahnende 243 aber so ausgestaltet, dass die Bewegung entweder zumindest eine Komponente hat, die zum Anschlussende 223 weist oder die Bewegung verläuft senkrecht zur Verbindungslinie 235.

Damit hat die Bewegungsbahn von dem Führungsbahnende 243 weg keine Komponente, die zum einen parallel zur Verbindungslinie 235 verläuft und zum anderen hin zu dem Kopplungsende 225 gerichtet ist. Mit anderen Worten muss sich der Zapfen 203, wenn er vom Führungsbahnende 243 hin zur Eintrittsöffnung 241 bewegt werden soll, zwangsweise weg vom Kopplungsende 225 bewegen. Um eine solche Bewegung auszuführen, muss das erste Verschlussteil 201, wenn der Verschluss in eine Gurtanordnung einer Orthese integriert ist, entgegen der Kraft bewegt werden, die durch die Vorspannung der Gurtanordnung auf den Verschluss ausgeübt wird. Dies führt dazu, dass sich der Verschluss selbst verriegelt. Es ist aber auch denkbar, dass die Bewegung des Zapfens 203 aus dem Führungsbahnende 243 heraus senkrecht zu der Verbindungslinie 235 zwischen dem Anschlussende 223 und dem Kopplungsende verläuft 225. Dann wirken, wenn der Verschluss unter Spannung steht, zumindest Reibungskräfte, die eine Bewegung des Zapfens 203 in der Führungsbahn 237 verhindern.

Wie außerdem aus Fig. 13 zu erkennen ist, verläuft die Verbindungslinie 235 im Übrigen von dem Anschlussende 223 mit der ersten Verbindungseinrichtung durch das Führungsbahnende 243 zum Kopplungsende 225, wobei die Verbindungslinie 235 auch durch die Mittelachse des Zapfens 203 bzw. des ersten Abschnitts 207 verläuft, wenn dieser sich im Führungsbahnende 243 befindet.

Die Weite X der Führungsbahn 237, und damit deren Abmessung senkrecht zur Tangente an deren Verlauf, entspricht dem Durchmesser des ersten Abschnitts 207 des ersten Verschlussteils 201 mit Ausnahme eines Bereichs zwischen Rastvorsprüngen 245, in denen die Weite reduziert ist. Allerdings ist es auch denkbar, dass die Weite der Führungsbahn 237 nicht über deren gesamte Länge den Abmessungen des ersten Abschnitts 207 entspricht, sondern beabstandet von dem Führungsbahnende 243 deutlich größer ausgebildet ist. Auf jeden Fall müssen aber die Abmessungen des zweiten Abschnitts bzw. des zweiten Vorsprungs 209 senkrecht zur Erstreckungsrichtung des Zapfens 203 größer sein als die Abmessung der Führungsbahn 237 am Führungsbahnende 243, sodass der zweite Abschnitt 209 eine Bewegung des Zapfens 203 relativ zu dem zweiten Verschlussteil 221 senkrecht zur Verschlussebene begrenzt. Unter dem Begriff des Entsprechens der Abmessungen ist in diesem Zusammenhang zu verstehen, dass der erste Abschnitt 207 und der Rand der Führungsbahn 237 im Führungsbahnende 243 nicht derart eng aneinander anliegen sollen, dass ein Verschieben des Zapfens 203 gegenüber dem zweiten Verschlussteil 221 nicht mehr möglich ist.

Die Rastvorsprünge 245 dienen dazu, das erste Verschlussteil 201 in einer Position zu verriegeln, in der es sich am Führungsbahnende 243 befindet. Dadurch muss bei diesem Ausführungsbeispiel das erste Verschlussteil 201 mit dem Zapfen 203 an den Vorsprüngen 245 vorbei bewegt werden, wobei diese entgegen einer elastischen Gegenkraft, die sich aus der Elastizität des ebenen Abschnitts 233 ergibt, aus der Führungsbahn 237 weg bewegt werden müssen.

Dadurch, dass der Zapfen 203 im Bereich des ersten Abschnitts 207 einen kreisförmigen Querschnitt hat und der Durchmesser der Weite X der Führungsbahn entspricht, kann das erste Verschlussteil 201 gegenüber dem zweiten Verschlussteil 221 verschwenkt werden.

Wie des Weiteren aus Fig. 13 zu erkennen ist, ist die Führungsbahn 237 derart ausgestaltet, dass sie den ersten Abschnitt 207 des Zapfens 203 aufnehmen und zwischen der Eintrittsöffnung 241 und dem Führungsbahnende 243 führen kann. Außerdem ist zu erkennen, dass die Eintrittsöffnung 241, die am Rand des ebenen Abschnitts 233 ausgebildet ist, derart angeordnet ist, dass deren Projektion auf die Verbindungslinie 235 weiter entfernt von dem Kopplungsende 225 ist als die Projektion des Führungsbahnendes 243 auf die Verbindungslinie 235. Somit wirkt auch an der Eintrittsöffnung 241 eine Kraft auf das erste Verschlussteil 201, wenn der Verschluss in eine Gurtanordnung integriert ist.

In dem ebenen Abschnitt 233 des zweiten Verbindungsteils ist schließlich eine zweite Verbindungseinrichtung für ein Gurtende in Form eines weiteren Schlitzes 247 ausgebildet, sodass das zweite Verschlussteil 221 mit einem zweiten Gurtende, das durch den weiteren Schlitz 247 geführt wird, verbunden werden kann. Dabei ist der weitere Schlitz 247 auf einer Seite der Verbindungslinie 235.

Wie außerdem aus Fig. 13 zu erkennen ist, ist die Eintrittsöffnung 241 der Führungsbahn 237 auf einer ersten Seite der Verbindungslinie 235 angeordnet, während die zweite Verbindungseinrichtung in Form des weiteren Schlitzes 247 auf einer gegenüberliegenden zweiten Seite angeordnet ist.

Wenn die Verschlussteile 201, 221 dieses Ausführungsbeispiels miteinander verbunden werden sollen und sie jeweils mit Gurtenden einer Gurtanordnung verbunden sind, muss das zweite Verbindungsteil 221 zunächst entgegen der Rückstellkräfte, die von der Gurtanordnung erzeugt werden, so weit hin zu dem ersten Verschlussteil 201 bewegt werden, dass der Zapfen 203 seitlich durch die Eintrittsöffnung 241 in die Führungsbahn 237 in dem ebenen Abschnitt 233 bewegt werden kann. Anschließend bewegt sich der Zapfen 203, in diesem Ausführungsbeispiel aufgrund der einheitlichen Krümmungsrichtung, entlang der Führungsbahn 237 und muss gegebenenfalls durch Drücken an den Vorsprüngen 245 vorbei bewegt werden, bis er das Führungsbahnende 243 erreicht. In dieser Position wird er, da dies der dem Kopplungsende 225 nächstgelegene Punkt der Führungsbahn 237 ist, aufgrund der Rückstellkräfte gehalten.

Wie aus den Fig. 16 bis 24 zu erkennen ist, ist das dritte Ausführungsbeispiel einer Orthese, in der zuvor beschriebene selbstständig erfinderische Verschluss integriert werden kann, zur Immobilisierung eines Schultergelenks vorgesehen und weist eine Gurtanordnung mit einem Zirkulargurt 251 auf, der ein erstes und ein zweites Ende 253, 255 hat (siehe Fig. 16 und 17) .

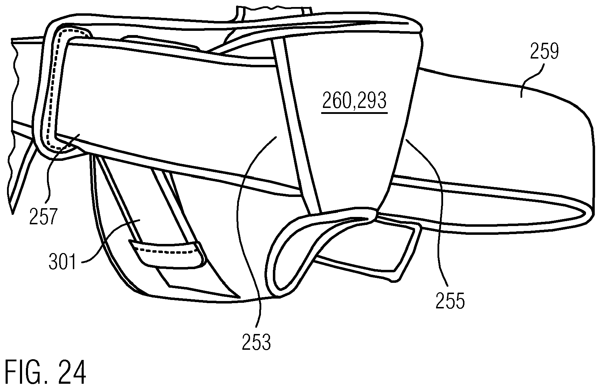

Der Zirkulargurt 251 setzt sich aus einem sich von dem ersten Ende 253 erstreckenden und anterior über den Patienten verlaufenden Anteriorabschnitt 257 und einem Posteriorabschnitt 259 zusammen, der von dem zweiten Ende 255 posterior und horizontal um den Patienten umläuft. Dabei sind bei diesem Ausführungsbeispiel das erste und das zweite Ende 253, 255 des Zirkulargurts 251 in der Weise miteinander verbunden, dass sie an einer Aufnahme 260 für den Ellenbogen benachbart zueinander befestigt sind, wie dies insbesondere in Fig. 24 gezeigt ist. Bei diesem Ausführungsbeispiel bildet das erste Ende 253 des Zirkulargurts 251 mit der Aufnahme 260 einen ersten Gurtabschnitt, der einen Armabschnitt 261 des Arms 263 des Patienten teilweise umschlingt, der sich von dem ruhigzustellenden ersten Schultergelenk 265 erstreckt.

Dabei wird unter dem Begriff "Armabschnitt" im Zusammenhang mit der vorliegenden Erfindung der Bereich des von dem verletzten ersten Schultergelenk 265 ausgehenden Arms 263 verstanden, der den Ellenbogen selbst sowie die dazu benachbarten Bereiche umfasst.

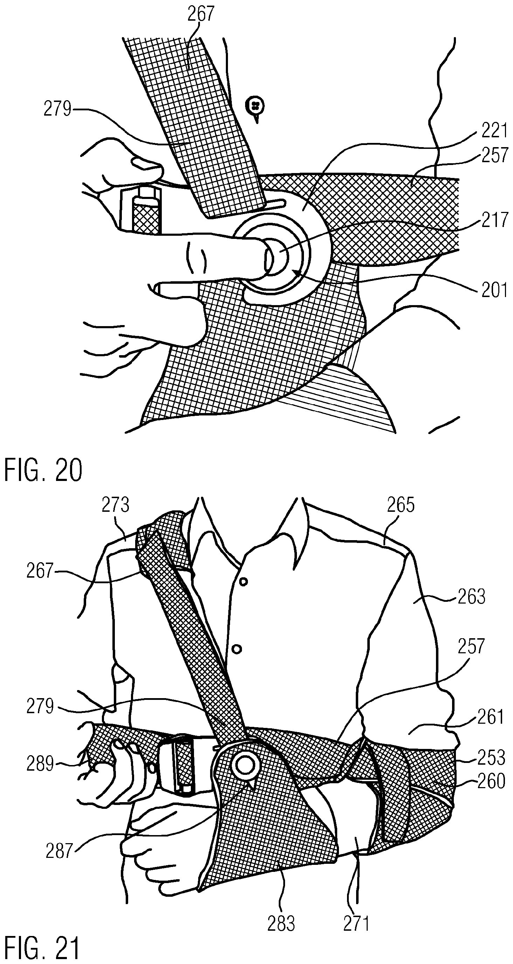

Aus den Fig. 16, 17 und 18 geht zudem hervor, dass bei dieser Orthese ein Schultergurt 267 vorgesehen ist, dessen erstes Ende 269 einen zweiten Gurtabschnitt bildet, wobei das erste Ende 269 mit der Aufnahme 260 verbunden ist und so den Armabschnitt 261 des Arms 263 des Patienten ebenfalls darüber umschlingt. Dabei geht das erste Ende 269 am Unterarm 271 benachbart zu dem Ellenbogen in die Aufnahme 260 über. Der Schultergurt 269 erstreckt sich posterior und diagonal über den Rücken des Patienten zu der dem ersten Schultergelenk 265 contralateralen Schulter 273. Wie die Fig. 16 und 21 zeigen, verläuft der Schultergurt 267 von der contralateralen Schulter 273 in vertikaler Richtung anterior zu dem Zirkulargurt 251 und ist dort in einem ersten Verbindungspunkt mit diesem über die Verschlussteile 201, 221 verbunden.

Wie den Fig. 16 bis 24 außerdem zu entnehmen ist, ist der Verschluss mit den Verschlussteilen 201, 221 in geschlossenem Zustand der Orthese im Bereich eines virtuellen Knotenpunkts 275 angeordnet (siehe Fig. 16).

Dabei ist das zweite Verschlussteil 221 mit dem zweiten Ende 279 des Schultergurts 267 in der Weise verbunden, dass das zweite Ende 279 durch den weiteren Schlitz 247 gezogen und das zweite Verschlussteil 221 damit entlang des Schultergurts 267 verschoben werden kann. Zum Verschieben des zweiten Endes 279 des Schultergurts 267 muss zunächst eine Klettverbindung geöffnet werden, und dann kann das zweite Ende 279 mehr oder weniger weit durch den weiteren Schlitz 247 gezogen werden. Außerdem ist das zweite Verschlussteil 221 mit dem Posteriorabschnitt 259 des Zirkulargurts 251 verbunden, wobei das zweite Verschlussteil 221 ebenfalls in dessen Längsrichtung verschiebbar ist. Hierzu ist der Posteriorabschnitt 259 in der schon beschriebenen Weise durch den ersten und zweiten Schlitz 29, 231 sowie um den Steg 227 geführt.

Mittels des zweiten Verschlussteils 221 sind somit das zweite Ende 279 des Schultergurts 267 und der Zirkulargurt 251 bzw. dessen Posteriorabschnitt 259 miteinander verbunden. Ferner ist dadurch, dass das zweite Verschlussteil 221 verschiebbar an dem Zirkulargurt 251 und dem Schultergurt 267 angebracht ist, ein erster Verbindungspunkt, an dem der Schultergurt 267 und der Zirkulargurt 251 miteinander verbunden sind, entlang des Zirkulargurts 251 und entlang des Schultergurts 267 verschiebbar.

Das erste Verschlussteil 201 ist an dem freien Ende 281 des Anteriorabschnitts 257 in der Weise befestigt, dass der Flansch 205 mit dem freien Ende 281 vernäht ist. Es ist aber auch denkbar, dass der Flansch 205 mit dem freien Ende 281 verklebt oder verschweißt ist.

Der erste Abschnitt 207 des Zapfens 203 kann in die Eintrittsöffnung 241 eingeschoben und entlang der Führungsbahn 237 bis zum Führungsbahnende 243 bewegt werden, um den aus den Verschlussteilen 201, 221 gebildeten Verschluss zu schließen. Dabei ist zu erkennen, dass sowohl in der geschlossenen als auch in der geöffneten Stellung der Schultergurt 267 und der Posteriorabschnitt 259 miteinander verbunden sind.

Fig. 16 und 24 zeigen ferner, dass ein Haltegurt 283 vorgesehen ist, dessen erstes Ende 285 mit dem freien Ende 281 des Anteriorabschnitts 257 verbunden ist. Das zweite Ende 287 des Haltegurts 283 kann lösbar mit dem ersten Verbindungsteil 201 verbunden werden, indem eine Aussparung 288 in dem zweiten Ende 287 in den Bereich des Hinterschnitts 213 gedrückt und so an dem freien Ende 215 des Zapfens 203 befestigt wird, sodass der Haltegurt 283 eine Schlaufe bildet, in die das Handgelenk des Unterarms 271, der von der dem ersten Schultergelenk 265 ausgeht, aufgenommen werden kann (siehe Fig. 10). Wie weiter aus Fig. 13 zu erkennen ist, verlaufen an dem Haltegurt 283 senkrecht zu dessen Erstreckungsrichtung Verstärkungsstreben 291, die ein Abknicken des Haltegurts 283 verhindern, sodass ein vom Haltegurt 283 aufgenommenes Handgelenk ebenfalls nicht abknicken kann.

Durch die am Anschlussende 223 des zweiten Verschlussteils 221 vorgesehenen zwei Schlitze 229, 231 und den dazwischen angeordneten Steg 227 ist eine Fixiervorrichtung vorgesehen, um die Lage des ersten Verbindungspunkts, an dem der Schultergurt 267 und der Posteriorabschnitt 259 miteinander verbunden sind, entlang des Posteriorabschnitts 259 festzulegen. Diese Fixiervorrichtung ist bei dem zuvor beschriebenen Aufbau derart ausgestaltet, dass der erste Verbindungspunkt durch Ziehen an dem vom zweiten Ende 255 des Zirkulargurts 251 bzw. des Posteriorabschnitts 259 entfernten Posteriorabschnittende 289 hin zu dem zweiten Ende 255 verschoben werden kann, während eine Bewegung des ersten Verbindungspunkts hin zum Posteriorabschnittende 289 durch Ziehen an dem zweiten Ende 255 blockiert wird. Dies bedeutet, dass der Posteriorabschnitt 259 durch Ziehen an dessen Ende 289 durch die Schlitze 229, 231 hindurch durch das Anschlussende 223 gezogen werden kann, während eine Bewegung des Posteriorabschnitts 259 in entgegengesetzter Richtung blockiert ist.

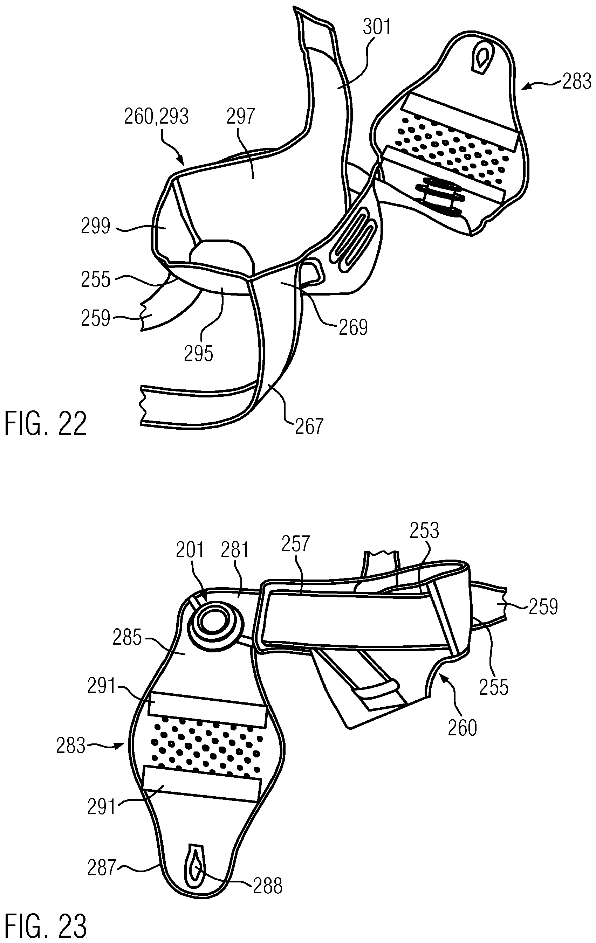

Wie schließlich den Fig. 22 bis 24 zu entnehmen ist, weist die Aufnahme 260 für den Ellenbogen eine Einrichtung auf, um deren Weite einstellen zu können. Die Aufnahme des Ellenbogens hat einen Hauptkörper 293, der eine am Körper des Patienten anliegende Innenfläche 295, eine vom Körper weg angeordneten Außenfläche 297 und eine außen am Oberarm anliegende Seitenfläche 299 umfasst. Dabei sind die Innenfläche 295 und die Außenfläche 297 an einer Kante, die am Unterarm anliegt, miteinander verbunden. An der Außenfläche 297 ist an der entfernt zu der Verbindung mit der Innenfläche 295 gelegenen Kante ein Verstellgurt 301 befestigt, der durch zwei Öffnungen 303 in der Innenfläche und über den Unterarm geführt und wiederum lösbar an der Außenfläche 297 befestigt werden kann. Durch den Verstellgurt 301 ist es möglich, die Weite der Aufnahme 260 an die Bedürfnisse des Patienten anzupassen.

Das zuvor beschriebene dritte Ausführungsbeispiel einer Orthese wird nun wie folgt angelegt.

Zuerst wird der Armabschnitt 261 in der mit den Enden 253, 269 des Zirkulargurts 251 und des Schultergurts 267 verbundenen Aufnahme 260 aufgenommen, sodass diese dann den Armabschnitt 261 über die Aufnahme 260 umschlingen. Dabei ist der Verstellgurt 301 über den Unterarm des Patienten geführt.

Da das zweite Ende 279 des Schultergurts 267 und der Posteriorabschnitt 259 über das zweite Verschlussteil 221 auch in der geöffneten Stellung des Verschlusses miteinander verbunden sind, bilden der Posteriorabschnitt 259 und der Schultergurt 267 zunächst eine Schlaufe, in die die zum ersten Schultergelenk 265 contralaterale Schulter 273 aufgenommen werden kann bzw. der Patient muss lediglich diese Schlaufe über die contralaterale Schulter 273 hängen.

Anschließend wird der aus den Verschlussteilen 201, 221 gebildete Verschluss in der Weise geschlossen, dass der Zapfen 203 in die Eintrittsöffnung 241 eingesetzt wird und dieser dann aufgrund der am freien Ende 281 des Anteriorabschnitts 257 wirkenden Kräfte zum Führungsbahnende 243 gezogen wird, wobei hierbei allerdings die Rastvorsprünge 245 entgegen der auf sie wirkenden elastischen Gegenkräfte aus der Führungsbahn 237 gedrückt werden müssen. Dabei zeigt insbesondere Fig. 20, dass die Vertiefung 217 im freien Ende 215 des Zapfens 203 ein Verschließen des Verschlusses mit einer Bewegung des Zapfens 203 entlang der Führungsbahn 237 mit nur einer Hand ermöglicht.

Wenn sich der Zapfen 203 am Führungsbahnende 243 befindet, wird dieser aufgrund des beschriebenen Verlaufs der Führungsbahn 237 und der entlang der Verbindungslinie wirkenden Kraft, die sich aus der Vorspannung in dem Zirkulargurt 251 und dem Schultergurt 267 ergibt, am Führungsbahnende 243 gehalten.

Schließlich kann dann der Zirkulargurt 251 zusätzlich durch Ziehen an dem Posteriorabschnittende 289 gespannt werden, wobei das zweite Verschlussteil 221 in der zuvor beschriebenen Weise ausgestaltet ist, sodass eine Rückbewegung des Posteriorabschnitts 259 nicht möglich ist. Danach ergibt sich die in Fig. 16 dargestellte Situation.

Schließlich kann der Patient den Unterarm 271 in der Weise fixieren, dass der Haltegurt 283 um den Unterarm geschlungen wird, wobei das zweite Ende 287 des Haltegurts 283 am Zapfen 203 des ersten Verschlussteils 201 befestigt wird (siehe Fig. 21) .

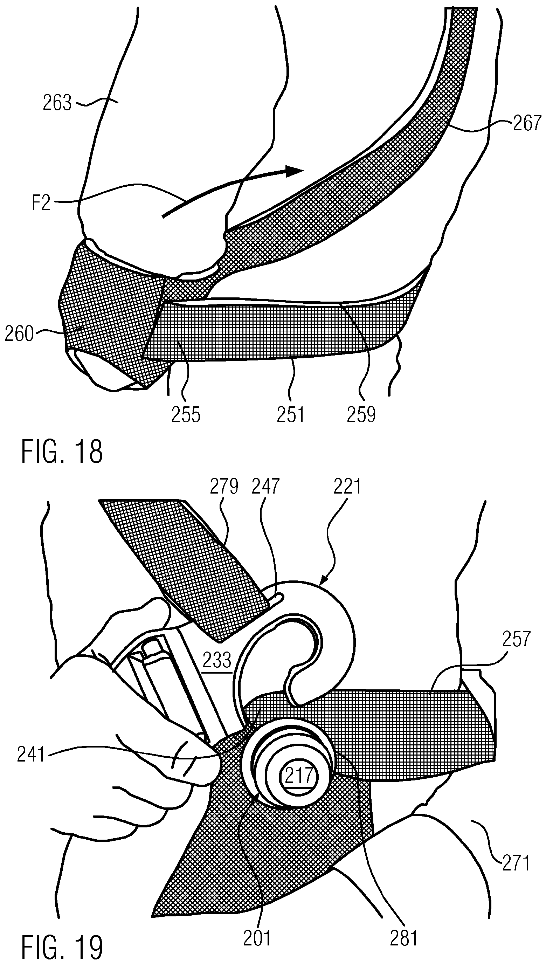

Bei zuvor beschriebenen Orthese sind demnach ein erster und ein zweiter Gurtabschnitt, nämlich das erste Ende 253 des Zirkulargurts 251 und das erste Ende 269 des Schultergurts 267, vorgesehen, die an dem Armabschnitt 261 in der Weise angreifen, dass sie ihn zumindest teilweise mit Hilfe der Aufnahme 260 umschlingen, sodass durch Zug in der Erstreckungsrichtung der Gurtabschnitte der Armabschnitt 261 mit einer ersten und einer zweiten Kraft beaufschlagt wird. Der erste Gurtabschnitt bzw. das erste Ende 253 des Zirkulargurts 251 übt im angelegten Zustand der Orthese, in der der von dem verletzten Schultergelenk 265 ausgehende Arm 263 seitlich bzw. frontal am Körper anliegt, eine Kraft (F1; siehe Fig. 16) auf den Armabschnitt 261 aus, die zu dem Körper hin gerichtet ist und die vorzugsweise im Wesentlichen parallel zu der Frontalebene des Patienten verläuft, aber wenigstens eine erste Komponente hat, die horizontal und parallel zur Frontalebene verläuft. Dadurch wird aufgrund des ersten Gurtabschnitts der Armabschnitt 261 an den Körper herangezogen, sodass die erste Kraftkomponente adduzierend auf den ersten Arm 263 wirkt. Gleichzeitig übt der zweite Gurtabschnitt, d.h. das erste Ende 269 des Schultergurts 267 eine zweite Kraft (F2; siehe Fig. 18) auf den Armabschnitt 261 aus, die wenigstens eine Komponente hat, die horizontal und parallel zur Sagittalebene und damit senkrecht zu der ersten Komponente verläuft und nach posterior gerichtet ist, sodass eine leichte Retroversion des Arms 263 bewirkt wird. Aufgrund dieser Kombination der zwei Kraftkomponenten wird der Arm 263 zuverlässig am Körper fixiert.

Außerdem ergibt sich der Vorteil, dass das erste, zu immobilisierende Schultergelenk 265 sowohl bei gestrecktem als auch bei gebeugtem Ellenbogen immobilisiert ist.

Des Weiteren zeigt sich, dass der Verschluss aus den beiden eingangs beschriebenen Verschlussteilen 201, 221 für den Patienten mit dem großen Vorteil verbunden, dass allein durch deren Lösen, also das Überführen von der geschlossenen in die geöffnete Stellung, die Wirkung beider Kräfte F1, F2 auf den Armabschnitt 261 aufgehoben wird, sodass der von dem verletzten Schultergelenk 265 ausgehende Arm 263 sofort freigegeben ist. Umgekehrt kann allein durch das Schließen des Verschlusses 201, 221 sofort die Wirkung der beiden Kräfte F1, F2 erreicht werden, ohne dass weitere Maßnahmen durch den Patienten erforderlich sind. Dies vereinfacht das Anlegen erheblich.

Allgemein umfasst die zuvor beschriebene Orthese somit eine Zirkulargurtanordnung 251, 257, 259, die ausgestaltet ist, zirkular und horizontal um einen Patienten umzulaufen, und die einen ersten und einen zweiten Abschnitt in Form des Posterior- und des Anteriorabschnitts 257, 259 aufweist, die vorgesehen sind, an einem Knotenpunkt 275 lösbar verbunden zu werden. Ferner weist diese Orthese eine Schultergurtanordnung 267 auf, die ein erstes mit der Zirkulargurtanordnung 251, 257, 259 verbundenes Ende 269 und ein zweites Ende aufweist und die angepasst ist, von Posterior nach Anterior über eine Schulter des Patienten hin zu dem Knotenpunkt 275 zu verlaufen. Am Knotenpunkt 275 ist der erste Abschnitt der Zirkulargurtanordnung 251, 257, 259, nämlich der Anteriorabschnitt 257, mit der Verbindungseinrichtung des ersten Verschlussteils 201 verbunden ist, während der zweite Abschnitt, nämlich der Posteriorabschnitt 259 mit der Verbindungseinrichtung 227, 229, 231 des zweiten Verschlussteils 221 verbunden ist. Schließlich ist die Schultergurtanordnung 267 mit der weiteren Verbindungseinrichtung 247 des zweiten Verschlussteils 221 verbunden ist.

Dabei ist durch den Aufbau der Verschlussteile 201, 221 gegeben, dass der Verschluss in einfacher Weise, ggf. nur mit einer Hand, verschlossen werden kann und er sich allein schon aufgrund des Verlaufs der Führungsbahn 237 in dieser Stellung hält. Damit kann eine mit dem Verschluss versehene Orthese auch von einem in seiner Bewegungsfreiheit eingeschränkten Patienten angelegt und verschlossen werden. Dies gilt nicht nur für die zuvor beschriebene Orthese zum Fixieren einer Schulter, sondern der Verschluss kann insbesondere auch bei anderen Orthesen, die eine Zirkulargurt- und eine Schultergurtanordnung aufweisen, wie beispielsweise Armabduktionskissen oder dgl., gewinnbringend eingesetzt werden.

## Patentansprüche

1. Orthese zur Immobilisierung eines ersten der Schultergelenke (15, 115, 265) eines Patienten,
mit einer Gurtanordnung, die angepasst ist, an einem Armabschnitt (11, 111, 261) am Ellenbogen oder benachbart zum Ellenbogen des sich von dem ersten Schultergelenk (15, 115, 265) aus ersteckenden Arms (13, 113, 263) anzugreifen, um den Ellenbogen frontal am Körper des Patienten zu fixieren,
wobei die Gurtanordnung einen ersten Gurtabschnitt (3, 103, 253) aufweist, der vorgesehen ist, den Armabschnitt (11, 111, 261) zumindest teilweise zu umschlingen,
wobei der erste Gurtabschnitt (3, 103, 253) vorgesehen ist, anterior entlang des Körpers des Patienten vom Armabschnitt (11) weg zu verlaufen, und
wobei der erste Gurtabschnitt (3, 103, 253) durch das erste Ende eines Zirkulargurts (1, 101, 251) der Gurtanordnung gebildet wird,
wobei der Zirkulargurt (1, 101, 251) angepasst ist, zirkular und horizontal um den Patienten umzulaufen und
wobei das zweite Ende (5, 105, 255) des Zirkulargurts (1, 101, 251) an dem Armabschnitt (11) angreift,
wobei der erste Gurtabschnitt (3, 103, 253) derart in die Gurtanordnung eingebunden ist, dass im angelegten Zustand der Orthese der erste Gurtabschnitt (3, 103, 253) eine erste Kraft (F1) auf den Armabschnitt (11, 111, 261) ausübt, die zumindest eine parallel zur Frontalebene des Patienten und horizontal verlaufende erste Komponente hat,
wobei die erste Komponente vom Armabschnitt (11, 111, 261) zum Körper hin weist, sodass die erste Kraft (F1) adduzierend auf den Arm (13, 113, 263) wirkt,
wobei die Gurtanordnung einen zweiten Gurtabschnitt aufweist, der vorgesehen ist, den Armabschnitt (11, 111, 261) zumindest teilweise zu umschlingen, und
wobei der zweite Gurtabschnitt derart in die Gurtanordnung eingebunden ist, dass im angelegten Zustand der Orthese der zweite Gurtabschnitt eine zweite Kraft (F2) auf den Armabschnitt (11) ausübt, die zumindest eine parallel zu der Sagittalebene des Patienten und horizontal verlaufende zweite Komponente hat,
wobei die zweite Komponente nach posterior gerichtet ist, sodass die zweite Kraft eine Retroversion des ersten Arms (13, 113, 263) bewirkt.

2. Orthese nach Anspruch 1, mit einer Verschlusseinrichtung (27, 127, 201, 221), die eine geöffnete und eine geschlossene Stellung hat,
wobei die Gurtanordnung derart ausgestaltet ist, dass in der geschlossenen Stellung die erste Kraft (F1) und die zweite Kraft (F2) auf den Armabschnitt (11, 111, 261) ausgeübt werden und dass in der geöffneten Stellung weder die erste Kraft (F1) noch die zweite Kraft (F2) auf den Armabschnitt (11, 111, 261) wirken.

3. Orthese nach Anspruch 1 oder 2, wobei das vom ersten Ende (3, 253) des Zirkulargurts (1) entfernte zweite Ende (5, 255) mit dessen erstem Ende (3, 253) verbunden ist und
wobei der zweite Gurtabschnitt an einem ersten Ende (19, 269) eines Schultergurts (17, 267) der Gurtanordnung ausgebildet ist, der angepasst ist, von dem Armabschnitt (11, 261) posterior zu der zu dem ersten Schultergelenk (15, 265) contralateralen Schulter (23, 273) des Patienten zu verlaufen und von der contralateralen Schulter (23, 273) anterior zu dem Zirkulargurt (1, 9, 251), und
wobei das von dem ersten Ende (19, 269) des Schultergurts entfernte zweite Ende (29, 279) auf der anterioren Seite des Patienten in einem ersten Verbindungspunkt mit dem Zirkulargurt (1, 9, 251) verbunden ist.

4. Orthese nach Anspruch 1 oder 2, wobei der zweite Gurtabschnitt an dem zweiten Ende (105) des Zirkulargurts (101) vorgesehen ist,
wobei die Gurtanordnung einen Schultergurt (117) aufweist, der in einem ersten Verbindungspunkt, der im angelegten Zustand der Orthese auf der anterioren Seite des Körpers des Patienten liegt, und in einem zweiten Verbindungspunkt, der im angelegten Zustand der Orthese auf der posterioren Seite des Körpers des Patienten liegt, mit dem Zirkulargurt (101, 109) verbunden ist.

5. Orthese nach Anspruch 4, wobei der zweite Verbindungspunkt, an dem der Zirkulargurt (101, 109) und der Schultergurt (117) miteinander verbunden sind, entlang des Zirkulargurts (101, 109) verschiebbar ist.

6. Orthese nach einem der Ansprüche 3 bis 5, wobei der erste Verbindungspunkt, an dem der Zirkulargurt (9, 109, 251) und der Schultergurt (17, 117, 267) miteinander verbunden sind, entlang des Zirkulargurts (9, 109, 251) verschiebbar ist.

7. Orthese nach einem der Ansprüche 3 bis 6, wobei der erste Verbindungspunkt, an dem der Zirkulargurt (9, 109, 251) und der Schultergurt (17, 117, 267) miteinander verbunden sind, entlang des Schultergurts (17, 117, 267) verschiebbar ist.

8. Orthese nach einem der Ansprüche 3 bis 7, wobei der Zirkulargurt (1, 101, 251) einen Anteriorabschnitt (7, 107, 257) aufweist, der sich von dem ersten Verbindungspunkt zu dem ersten Ende (3, 103, 253) des Zirkulargurts (1, 101, 251) erstreckt,
wobei der Zirkulargurt (1, 101, 251) einen Posteriorabschnitt (9, 109, 259) aufweist, der vorgesehen ist, horizontal und posterior um den Körper des Patienten zu verlaufen, der sich von einem Posteriorabschnittende (45, 145, 289) zu dem zweiten Ende (5, 105, 255) des Zirkulargurts (1, 101, 251) erstreckt und an dem der erste Verbindungspunkt angeordnet ist, und
wobei der Anteriorabschnitt (7, 107, 257) und der Posteriorabschnitt (9, 109, 259) lösbar miteinander verbunden sind.

9. Orthese nach einem der Ansprüche 3 bis 8, sofern abhängig von Anspruch 2, wobei die Verschlusseinrichtung (27, 127, 201, 221) an dem ersten Verbindungspunkt vorgesehen ist.

10. Orthese nach Anspruch 8 und 9, wobei die Verschlusseinrichtung (27, 127, 201, 221) derart ausgestaltet ist, dass in der geschlossenen Stellung der Verschlusseinrichtung (27, 127, 201, 221) der Anteriorabschnitt (7, 107, 257) und der Posteriorabschnitt (9, 109, 259) miteinander verbunden sind und dass in der geöffneten Stellung der Anteriorabschnitt (7, 107, 257) und der Posteriorabschnitt (9, 109, 259) voneinander gelöst sind.

11. Orthese nach Anspruch 10, wobei die Verschlusseinrichtung (27, 127, 201, 221) derart ausgestaltet ist, dass der Schultergurt (17, 117, 267) in der geöffneten und der geschlossene Stellung mit dem Posteriorabschnitt (9, 109, 259) verbunden ist.

12. Orthese nach Anspruch 10 oder 11, wobei die Verschlusseinrichtung (27, 127, 201, 221) eine Fixiervorrichtung zum Festlegen des ersten Verbindungspunkts entlang des Posteriorabschnitts (9, 109, 259) aufweist,
wobei die Fixiervorrichtung derart ausgestaltet ist, dass der erste Verbindungspunkt durch Ziehen an dem Posteriorabschnittende (45, 145, 289) hin zu dem zweiten Ende (5, 105, 255) des Zirkulargurts (1, 101, 251) verschoben werden kann, während eine Bewegung des ersten Verbindungspunkts hin Posteriorabschnittende (45, 145, 289) durch Ziehen an dem zweiten Ende (5, 105, 255) des Zirkulargurts (1, 101, 251) blockiert wird.

13. Orthese nach einem der Ansprüche 1 bis 12 mit einer Halteeinrichtung (37, 137, 283), die ausgestaltet ist, den Unterarm (21, 121, 271) des sich von dem ersten Schultergelenk (15, 115, 265) erstreckenden Arms (13, 113, 263) in dessen gebeugter Stellung zu halten.

14. Orthese nach Anspruch 13, wobei die Halteeinrichtung einen Haltegurt (37, 137, 283) aufweist, der mit einem ersten Ende (39, 285) mit der Gurtanordnung verbunden ist und der angepasst ist, den Unterarm (21, 271) des Arms (13, 263), der sich von dem ersten Schultergelenk (15, 265) erstreckt, im Bereich von dessen Handgelenk zu halten.

15. Orthese nach Anspruch 14, wobei der Haltegurt (37, 137) mit einem ersten Ende (39, 139) mit dem Schultergurt (17, 117) verbunden ist oder dessen erstes Ende (39, 139) durch den Schultergurt (17, 117) gebildet wird

16. Orthese nach Anspruch 15, wobei der Haltegurt (37, 137) angepasst ist, den Unterarm (21) des Arms (13), der sich von dem ersten Schultergelenk (15) erstreckt, zu umschlingen, und
wobei ein zweites Ende (41, 141) des Haltegurts (37, 137) lösbar mit dem Schultergurt (17, 117) verbunden werden kann.

17. Orthese nach einem der Ansprüche 1 bis 16, mit einer Unterarmauflage (43, 143), die angepasst ist, den Unterarm (21) mit der Hand des von dem ersten Schultergelenk (15) ausgehenden Arms (13) aufzunehmen.

## Claims

1. An orthosis for immobilizing a first one of the shoulder joints (15, 115, 265) of a patient,
having a strap arrangement which is designed to engage on an arm section (11, 111, 261) at the elbow or adjacent to the elbow of the arm (13, 113, 263) extending from the first shoulder joint (15, 115, 265) in order to fix the elbow frontally on the body of the patient,
wherein the strap arrangement has a first strap section (3, 103, 253) which is provided for at least partially looping around the arm section (11, 111, 261),
wherein the first strap section (3, 103, 253) is provided so as to run anteriorly along the body of the patient away from the arm section (11),
wherein the first strap section (3, 103, 253) is formed by the first end of a circular strap (1, 101, 251) of the strap arrangement,
wherein the circular strap (1, 101, 251) is designed to run circularly and horizontally around the patient, and
wherein the second end (5, 105, 255) of the circular strap (1, 101, 251) engages on the arm section (11),
wherein the first strap section (3, 103, 253) is incorporated into the strap arrangement such that, in the fitted state of the orthosis, the first strap section (3, 103, 253) exerts on the arm section (11, 111, 261) a first force (F1) which has at least a first component running parallel to the frontal plane of the patient and horizontally,
wherein the first component points from the arm section (11, 111, 261) toward the body, such that the first force (F1) has an adductive effect on the arm (13, 113, 263),
wherein the strap arrangement has a second strap section which is provided for at least partially looping around the arm section (11, 111, 261), and
wherein the second strap section is incorporated into the strap arrangement such that, in the fitted state of the orthosis, the second strap section exerts on the arm section (11) a second force (F2) which has at least a second component running parallel to the sagittal plane of the patient and horizontally,
wherein the second component is directed toward the posterior, such that the second force effects a retroversion of the first arm (13, 113, 263).

2. The orthosis as claimed in claim 1, having a fastener device (27, 127, 201, 221) which has an open and a closed position,
wherein the strap arrangement is designed such that, in the closed position, the first force (F1) and the second force (F2) are exerted on the arm section (11, 111, 261), and that, in the open position, neither the first force (F1) nor the second force (F2) act on the arm section (11, 111, 261).

3. The orthosis as claimed in claim 1 or 2, wherein the second end (5, 255), which is remote from the first end (3, 253) of the circular strap (1), is connected to the first end (3, 253) of said circular strap, and
wherein the second strap section is formed on a first end (19, 269) of a shoulder strap (17, 267) of the strap arrangement, which shoulder strap is designed to run posteriorly from the arm section (11, 261) to the contralateral shoulder (23, 273) of the patient in relation to the first shoulder joint (15, 265) and anteriorly from the contralateral shoulder (23, 273) to the circular strap (1, 9, 251), and
wherein the second end (29, 279), which is remote from the first end (19, 269) of the shoulder strap, is connected to the circular strap (1, 9, 251) at a first connecting point on the anterior side of the patient.

4. The orthosis as claimed in claim 1 or 2, wherein the second strap section is provided on the second end (105) of the circular strap (101),
wherein the strap arrangement has a shoulder strap (117) which is connected to the circular strap (101, 109) at a first connecting point, which in the fitted state of the orthosis is situated on the anterior side of the body of the patient, and at a second connecting point, which in the fitted state of the orthosis is situated on the posterior side of the body of the patient.

5. The orthosis as claimed in claim 4, wherein the second connecting point at which the circular strap (101, 109) and the shoulder strap (117) are connected to one another is displaceable along the circular strap (101, 109).

6. The orthosis as claimed in one of claims 3 to 5, wherein the first connecting point at which the circular strap (9, 109, 251) and the shoulder strap (17, 117, 267) are connected to one another is displaceable along the circular strap (9, 109, 251).

7. The orthosis as claimed in one of claims 3 to 6, wherein the first connecting point at which the circular strap (9, 109, 251) and the shoulder strap (17, 117, 267) are connected to one another is displaceable along the shoulder strap (17, 117, 267).

8. The orthosis as claimed in one of claims 3 to 7, wherein the circular strap (1, 101, 251) has an anterior section (7, 107, 257) which extends from the first connecting point to the first end (3, 103, 253) of the circular strap (1, 101, 251),
wherein the circular strap (1, 101, 251) has a posterior section (9, 109, 259) which is provided so as to run horizontally and posteriorly around the body of the patient, which extends from a posterior section end (45, 145, 289) to the second end (5, 105, 255) of the circular strap (1, 101, 251) and on which the first connecting point is arranged, and
wherein the anterior section (7, 107, 257) and the posterior section (9, 109, 259) are detachably connected to one another.

9. The orthosis as claimed in one of claims 3 to 8, where dependent on claim 2, wherein the fastener device (27, 127, 201, 221) is provided at the first connecting point.

10. The orthosis as claimed in claim 8 and 9, wherein the fastener device (27, 127, 201, 221) is designed such that, in the closed position of the fastener device (27, 127, 201, 221), the anterior section (7, 107, 257) and the posterior section (9, 109, 259) are connected to one another, and that, in the open position, the anterior section (7, 107, 257) and the posterior section (9, 109, 259) are detached from one another.

11. The orthosis as claimed in claim 10, wherein the fastener device (27, 127, 201, 221) is designed such that the shoulder strap (17, 117, 267) is connected to the posterior section (9, 109, 259) in the open position and in the closed position.

12. The orthosis as claimed in claim 10 or 11, wherein the fastener device (27, 127, 201, 221) has a fixing device for fixing the first connecting point along the posterior section (9, 109, 259),
wherein the fixing device is designed such that the first connecting point can be displaced toward the second end (5, 105, 255) of the circular strap (1, 101, 251) by pulling on the posterior section end (45, 145, 289), whereas a movement of the first connecting point towards the posterior section end (45, 145, 289) as a result of a pulling action on the second end (5, 105, 255) of the circular strap (1, 101, 251) is blocked.

13. The orthosis as claimed in one of claims 1 to 12, having a holding device (37, 137, 283) which is designed to hold the lower arm (21, 121, 271) of the arm (13, 113, 263) extending from the first shoulder joint (15, 115, 265) in its flexed position.

14. The orthosis as claimed in claim 13, wherein the holding device has a holding strap (37, 137, 283) which is connected by way of a first end (39, 285) to the strap arrangement and which is designed to hold the lower arm (21, 271) of the arm (13, 263) extending from the first shoulder joint (15, 265) in the region of the wrist thereof.

15. The orthosis as claimed in claim 14, wherein the holding strap (37, 137) is connected by way of a first end (39, 139) to the shoulder strap (17, 117), or the first end (39, 139) of said holding strap is formed by the shoulder strap (17, 117).

16. The orthosis as claimed in claim 15, wherein the holding strap (37, 137) is designed to loop around the lower arm (21) of the arm (13) extending from the first shoulder joint (15), and
wherein a second end (41, 141) of the holding strap (37, 137) can be detachably connected to the shoulder strap (17, 117).

17. The orthosis as claimed in one of claims 1 to 16, having a lower arm support (43, 143) which is designed to receive the lower arm (21), with the hand, of the arm (13) extending from the first shoulder joint (15).

## Revendications

1. Orthèse pour l'immobilisation d'une première des articulations d'épaule (15, 115, 265) d'un patient,
avec une disposition de sangle qui est conçue pour s'accrocher à une portion de bras (11, 111, 261) sur le coude ou à proximité du coude du bras (13, 113, 263) s'étendant à partir de la première articulation d'épaule (15, 115, 265), afin de fixer le coude de manière frontale au corps du patient,
la disposition de sangle comprenant une première portion de sangle (3, 103, 253) qui est conçue pour entourer la portion de bras (11, 111, 261) au moins partiellement,
la première portion de sangle (3, 103, 253) étant conçue pour s'étendre de manière antérieure le long du corps du patient en s'éloignant de la portion de bras (11) et
la première portion de sangle (3, 103, 253) étant constituée de la première extrémité d'une sangle circulaire (1, 101, 251) de la disposition de sangle,
la sangle circulaire (1, 101, 251) étant conçue pour s'étendre autour du patient de manière circulaire et horizontale et
la deuxième extrémité (5, 105, 255) de la sangle circulaire (1, 101, 251) s'accrochant à la portion de bras (11),
la première portion de sangle (3, 103, 253) étant intégrée dans la disposition de sangle de façon à ce que, dans l'état posé de l'orthèse, la première portion de sangle (3, 103, 253) exerce sur la portion de bras (11, 11, 261) une première force (F1) qui comprend au moins une première composante parallèle au plan frontal du patient et s'étendant horizontalement,
la première composante étant orientée de la portion de bras (11, 111, 261) vers le corps de façon à ce que la première force (F1) agisse en adduction sur le bras (13, 113, 263),
la disposition de sangle comprenant une deuxième portion de sangle qui est conçue pour entourer au moins partiellement la portion de bras (11, 111, 261) et
la deuxième portion de sangle étant intégrée dans la disposition de sangle de façon à ce que, dans l'état posé de l'orthèse, la deuxième portion de sangle exerce sur la portion de bras (11) une deuxième force (F2) qui comprend au moins une deuxième composante parallèle au plan sagittal du patient et s'étendant horizontalement,
la deuxième composante étant orientée vers le côté postérieur, de façon à ce que la deuxième force provoque une rétroversion du premier bras (13, 113, 263).

2. Orthèse selon la revendication 1, avec un dispositif de fermeture (27, 127, 201, 221) qui présente une position ouverte et une position fermée,
la disposition de sangle étant conçue de façon à ce que, dans la position fermée, la première force (F1) et la deuxième force (F2) soient exercées sur la portion de bras (11, 111, 261) et à ce que, dans la position ouverte, ni la première force (F1) ni la deuxième force (F2) n'agissent sur la portion de bras (11, 111, 261).

3. Orthèse selon la revendication 1 ou 2, la deuxième extrémité (5, 255) éloignée par la première extrémité (3, 253) de la sangle circulaire (1) étant reliée avec cette première extrémité (3, 253) et
la deuxième portion de sangle (17, 267) étant réalisée au niveau d'une première extrémité (19, 269) d'une sangle d'épaule (17, 267) de la disposition de sangle, qui est conçue pour s'étendre de la portion de bras (11, 261), de manière postérieure, vers l'épaule (23, 273) controlatérale à la première articulation d'épaule (15, 265) du patient et de l'épaule controlatérale (23, 273), de manière antérieure, vers la sangle circulaire (1, 9, 251) et
la deuxième extrémité (29, 279) éloignée par la première extrémité (19, 269) de la sangle d'épaule est reliée, sur le côté antérieur du patient, au niveau d'un premier point de liaison avec la sangle circulaire (1, 9, 251).

4. Orthèse selon la revendication 1 ou 2, la deuxième portion de sangle étant prévue sur la deuxième extrémité (105) de la sangle circulaire (101),
la disposition de sangle comprenant une sangle d'épaule (117) qui est reliée, au niveau d'un premier point de liaison, qui se trouve, dans l'état posé de l'orthèse, sur le côté antérieur du corps du patient, et au niveau d'un deuxième point de liaison, qui se trouve, dans l'état posé de l'orthèse, sur le côté postérieur du corps du patient, avec la sangle circulaire (101, 109).

5. Orthèse selon la revendication 4, le deuxième point de liaison, au niveau duquel la sangle circulaire (101, 109) et la sangle d'épaule (117) sont reliées entre elles, peut être coulissé le long de la sangle circulaire (101, 109).

6. Orthèse selon l'une des revendications 3 à 5, le premier point de liaison, au niveau duquel la sangle circulaire (9, 109, 251) et la sangle d'épaule (17, 117, 267) sont reliées entre elles, peut être coulissé le long de la sangle circulaire (9, 109, 251).

7. Orthèse selon l'une des revendications 3 à 6, le premier point de liaison, au niveau duquel la sangle circulaire (9, 109, 251) et la sangle d'épaule (17, 117, 267) sont reliées entre elles, peut être coulissé le long de la sangle d'épaule (17, 117, 267).

8. Orthèse selon l'une des revendications 3 à 7, la sangle circulaire (1, 101, 251) comprenant une portion antérieure (7, 107, 257) qui s'étend du premier point de liaison vers la première extrémité (3, 103, 253) de la sangle circulaire (1, 101, 251),
la sangle circulaire (1, 101, 251) comprenant une portion postérieure (9, 109, 259), qui est conçue pour s'étendre de manière horizontale et postérieure autour du corps du patient, qui s'étend d'une extrémité de portion postérieure (45, 145, 289) vers la deuxième extrémité (5, 105, 255) de la sangle circulaire (1, 101, 251) et sur laquelle est disposé le premier point de liaison et
la portion antérieure (7, 107, 257) et la portion postérieure (9, 109, 259) étant reliées entre elles de manière amovible.

9. Orthèse selon l'une des revendications 3 à 8, dans la mesure où elles dépendent de la revendication 2, le dispositif de fermeture (27, 127, 201, 221) étant prévu sur le premier point de liaison.

10. Orthèse selon la revendication 8 et 9, le dispositif de fermeture (27, 127, 201, 221) étant conçu de façon à ce que, dans la position fermée du dispositif de fermeture (27, 127, 201, 221), la portion antérieure (7, 107, 257) et la portion postérieure (9, 109, 259) soient reliées entre elles et à ce que, dans la position ouverte, la portion antérieure (7, 107, 257) et la portion postérieure (9, 109, 259) soient détachées l'une de l'autre.

11. Orthèse selon la revendication 10, le dispositif de fermeture (27, 127, 201, 221) étant conçu de façon à ce que la sangle d'épaule (17, 117, 267) soit reliée, dans la position ouverte et dans la position fermée, avec la portion postérieure (9, 109, 259).

12. Orthèse selon la revendication 10 ou 11, le dispositif de fermeture (27, 127, 201, 221) comprenant un dispositif de fixation pour la fixation du premier point de liaison le long de la portion postérieure (9, 109, 259),
le dispositif de fixation étant conçu de façon à ce que le premier point de liaison puisse être coulissé par une traction sur l'extrémité de portion postérieure (45, 145, 289) en direction de la deuxième extrémité (5, 105, 255) de la sangle circulaire (1, 101, 251), tandis qu'un déplacement du premier point de liaison en direction de l'extrémité de portion postérieure (45, 145, 289) est bloquée par une traction sur la deuxième extrémité (5, 105, 255) de la sangle circulaire (1, 101, 251).

13. Orthèse selon l'une des revendications 1 à 12, avec un dispositif de maintien (37, 137, 283) qui est conçue pour maintenir l'avant-bras (21, 121, 271) du bras (13, 113, 263) s'étendant à partir de la première articulation d'épaule (15, 115, 265) dans sa position pliée.

14. Orthèse selon la revendication 13, le dispositif de maintien comprenant une sangle de maintien (37, 137, 283) qui est reliée avec une première extrémité (39, 285) avec la disposition de sangle, et qui est conçue pour maintenir l'avant (21, 271) du bas (13, 263) qui s'étend à partir de la première articulation d'épaule (15, 265), au niveau de son poignet.

15. Orthèse selon la revendication 14, la sangle de maintien (37, 137) étant reliée avec sa première extrémité (39, 139) avec la sangle d'épaule (17, 117) ou sa première extrémité (39, 139) est constituée par la sangle d'épaule (17, 117).

16. Orthèse selon la revendication 15, la sangle de maintien (37, 137) étant conçue pour entourer l'avant-bras (21) du bras (13), qui s'étend à partir de la première articulation d'épaule (15) et
une deuxième extrémité (41, 141) de la sangle de maintien (37, 137) pouvant être reliée de manière amovible avec la sangle d'épaule (17, 117).

17. Orthèse selon l'une des revendications 1 à 16, avec un appui d'avant-bras (43, 143) qui est conçue pour loger l'avant-bras (21) avec la main du bras (13) partant de la première articulation d'épaule (15).
